(19) Europäisches Patentamt
European Patent Office
Office européen des brevets

(11) EP 4 414 364 A1

(12) # EUROPEAN PATENT APPLICATION
published in accordance with Art. 153(4) EPC

(43) Date of publication:
**14.08.2024 Bulletin 2024/33**

(21) Application number: **22878615.8**

(22) Date of filing: **07.10.2022**

(51) International Patent Classification (IPC):
***C07D 403/04*** *(2006.01)* ***A01P 7/04*** *(2006.01)*
***A01N 43/653*** *(2006.01)*

(52) Cooperative Patent Classification (CPC):
**A01N 43/653; A01P 7/04; C07D 403/04**

(86) International application number:
**PCT/JP2022/037582**

(87) International publication number:
**WO 2023/058748 (13.04.2023 Gazette 2023/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.10.2021 JP 2021165983**

(71) Applicant: **Nihon Nohyaku Co., Ltd.**
**Tokyo 104-8386 (JP)**

(72) Inventors:
- **YAMASHITA Yudai**
  **Kawachinagano-shi, Osaka 586-0094 (JP)**
- **HARAYAMA Hiroto**
  **Kawachinagano-shi, Osaka 586-0094 (JP)**
- **NATSUSAKA Ryutaro**
  **Kawachinagano-shi, Osaka 586-0094 (JP)**

(74) Representative: **Carpmaels & Ransford LLP**
**One Southampton Row**
**London WC1B 5HA (GB)**

(54) **PYRIMIDINYL TRIAZOLE COMPOUND OR SALT THEREOF, PEST CONTROL AGENT CONTAINING SAID COMPOUND AS ACTIVE INGREDIENT, AND PEST CONTROL METHOD**

(57) The present invention addresses the problem of providing a novel agricultural/horticultural pest control agent that is safe for honeybees and is effective for the control of pests. Provided is a compound represented by formula (I) or a salt thereof.

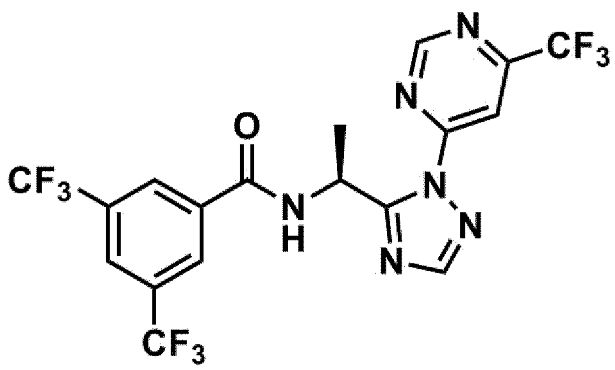

(I)

EP 4 414 364 A1

## Description

[Technical Field]

The present invention relates to a pyrimidinyl triazole compound or a salt thereof, an agrohorticultural pest control agent comprising said compound as an active ingredient and a method of controlling a pest.

[Background Art]

Patent document 1 describes that certain pyrimidinyl triazole compounds are useful as ectoparasite control agents for animals. Patent document 2 describes that said pyrimidinyl triazole compounds are useful as agrohorticultural pest control agents. Patent documents 3-24 describe that other pyrimidinyl triazole compounds are useful as agrohorticultural pest control agents or as ectoparasite control agents for animals.

[Prior Art References]

[Patent Document]

[Patent Document 1] WO2017/192385
[Patent Document 2] WO2019/170626
[Patent Document 3] WO2019/197468
[Patent Document 4] WO2019/201835
[Patent Document 5] WO2019/202077
[Patent Document 6] WO2019/206799
[Patent Document 7] WO2019/215198
[Patent Document 8] WO2020/002563
[Patent Document 9] WO2020/053365
[Patent Document 10] WO2020/079198
[Patent Document 11] WO2020/094363
[Patent Document 12] WO2020/182649
[Patent Document 13] WO2020/188014
[Patent Document 14] WO2020/188027
[Patent Document 15] WO2020/193341
[Patent Document 16] WO2020/212235
[Patent Document 17] WO2021/013719
[Patent Document 18] WO2021/013720
[Patent Document 19] WO2021/069567
[Patent Document 20] WO2021/069569
[Patent Document 21] WO2021/083936
[Patent Document 22] WO2021/099303
[Patent Document 23] WO2021/110891
[Patent Document 24] WO2021/122645

[Summary of Invention]

[Problem to be solved by the Invention]

In the production of useful crops in the agrohorticultural fields, damage caused by pests is enormous, and many effective agrohorticultural pest control agents have been marketed. However, due to the occurrence of insecticide-resistant pests against existing agents and the demand for agents with physical properties suitable for various labour-saving application methods, development of new agrohorticultural pest control agents is still one of the most important issues in the field of agrohorticulture. Furthermore, in recent years, safety for useful insects, especially honeybees, has become a major issue to be solved, and there is a need for pest control agents that are safe for honeybees and effective against agricultural and horticultural pests.

[Means for Solving the Problem]

The present inventors have conducted extensive research to develop a new pest control agent for agrohorticultural use that is safe for honeybees, and have found that the present inventive novel pyrimidinyl triazole compound or a salt thereof is safe for honeybees and completed the present invention.
Namely, the present invention relates to:

[1] A compound represented by formula (I) or a salt thereof:

[Chem 1]

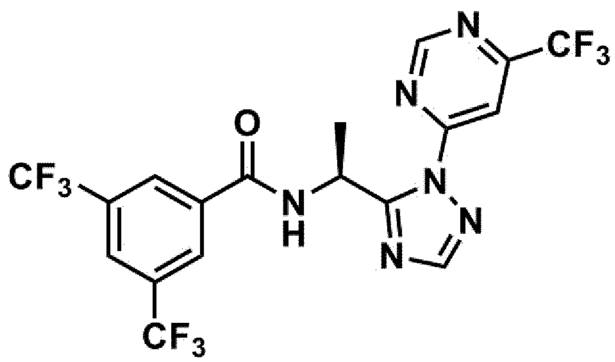

(I)
;

[2] An agrohorticultural pest control agent comprising the compound or a salt thereof described in [1] as an active ingredient;
[3] A method of use of an agrohorticultural pest control agent, characterized in that an effective amount of the agrohorticultural pest control agent described in [2] is treated on a target plant or soil; and
[4] A method of controlling an agrohorticultural pest, characterized in that an effective amount of the agrohorticultural pest control agent described in [2] is treated on a target plant or soil.

[Effect of Invention]

The present invention provides a pyrimidinyl triazole compound or a salt thereof that is safe for honeybees and has an excellent effect as an agrohorticultural pest control agent.

[Embodiment to Implement the Invention]

The present inventive pyrimidinyl triazole compound represented by formula (I) can be produced according to the production method disclosed in Patent Document 1, e.g., by the following production method, which is not limited thereto.

Production Method

[Chem 2]

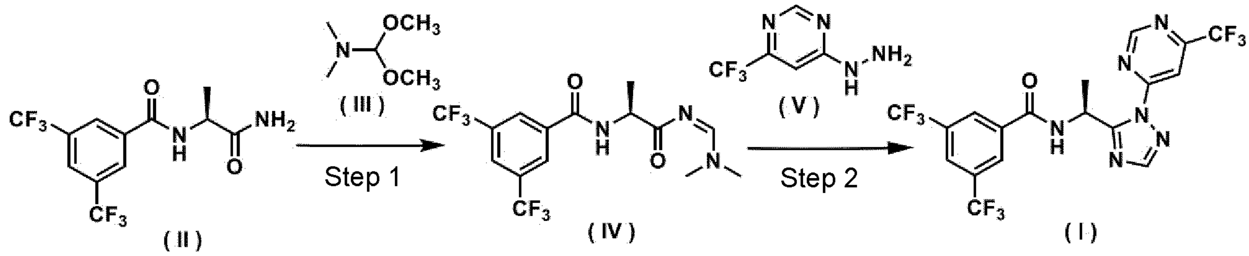


By reacting a compound represented by formula (II) with a dimethylformamide dimethylacetal represented by formula (III) as described in Patent Document 1 in the presence of an inert solvent, a compound represented by formula (IV) is formed (step 1), with or without isolating, by reacting the compound with the hydrazinopyrimidine compound represented by formula (V) in the presence of an acid catalyst and an inert solvent (step 2), the present inventive compound represented by formula (I) can be produced.

<Step 1>

Any inert solvent may be used as long as it does not significantly inhibit the progress of the reaction, e.g. linear or cyclic ethers such as diethyl ether, tetrahydrofuran, dioxane; esters such as ethyl acetate, butyl acetate; aromatic hydrocarbons such as benzene, toluene, xylene; amides such as dimethylformamide, N-methylacetamide, N,N-dimethylacetamide, N-methylpyrrolidone, hexamethylphosphoramide; halogenated hydrocarbons such as methylene chloride, chloroform, carbon tetrachloride; halogenated aromatic hydrocarbons such as chlorobenzene, dichlorobenzene; and other inert solvents can be cited, halogenated hydrocarbons being particularly preferred. These inert solvents can be used alone or in a mixture of two or more. The amount of the inert solvent used is not specifically limited as long as it is sufficient to dissolve the compound represented by formula (II) and can be selected in the range of 0.01 mol/L to 100 mol/L with regard to the compound represented by formula (II).

The reaction temperature may be selected from a range of about -20 to 150 °C, with a range of 20 °C to 100 °C being preferred. The reaction time is not constant depending on the reaction scale, reaction temperature and other factors, possibly being selected from a range of several minutes to about 48 hours. After completion of the reaction, the target product can be isolated from the reaction solution containing the compound represented by formula (IV) by conventional methods and purified using an appropriate purification method such as recrystallisation, distillation, column chromatography, etc., as necessary to produce the compound represented by formula (IV). The compound represented by formula (IV) can also be provided in step 2 without isolation. Since the reaction is an equimolar reaction, equal moles of each reactant may be used, and an excess of any of the reactants can also be used.

<Step 2>

Any acid catalyst can be used in this reaction as long as it promotes the reaction, e.g. organic acids such as formic acid, acetic acid, propionic acid and inorganic acids such as hydrochloric acid, sulphuric acid, phosphoric acid can be recited. The amount of acid catalyst used may be appropriately selected in the range of about 0.5 to 5 times mol to the compound represented by formula (IV), and the acid catalyst can also be used as a solvent.

Any inert solvent may be used as long as it does not significantly inhibit the progress of the reaction, e.g. linear or cyclic ethers such as diethyl ether, tetrahydrofuran, dioxane; esters such as ethyl acetate, butyl acetate; aromatic hydrocarbons such as benzene, toluene, xylene; amides such as dimethylformamide, N-methylacetamide, N,N-dimethylacetamide, N-methylpyrrolidone, hexamethylphosphoramide; nitriles such as acetonitrile, propionitrile; organic acids such as formic acid, acetic acid, propionic acid;
halogenated hydrocarbons such as methylene chloride, chloroform, carbon tetrachloride; halogenated aromatic hydrocarbons such as chlorobenzene, dichlorobenzene; and other inert solvents can be cited, cyclic ethers or organic acids being particularly preferred. These inert solvents can be used alone or in a mixture of two or more. The amount of the inert solvent used is not specifically limited as long as it is sufficient to dissolve the compound represented by formula (IV) and can be selected in the range of 0.01 mol/L to 100 mol/L with regard to the compound represented by formula (IV).

The reaction temperature may be selected from a range of about -20 to 150 °C, with a range of 20 °C to 100 °C being preferred. The reaction time is not constant depending on the reaction scale, reaction temperature and other factors, possibly being selected from a range of several minutes to about 48 hours. After completion of the reaction, the target product can be isolated from the reaction solution containing the compound represented by formula (I) by conventional methods and purified using an appropriate purification method such as recrystallisation, distillation, column

chromatography, etc., as necessary to produce the compound represented by formula (I). Since the reaction is an equimolar reaction, equal moles of each reactant may be used, and an excess of any of the reactants can also be used.

The salt of the present inventive compound represented by formula (I) include, for example,

inorganic salts such as hydrochloride, sulfate, nitrate, phosphate and so forth;
organic salts such as acetate, fumarate, malate, oxalate, methanesulfonate, benzenesulfonate, paratoluenesulfonate and so forth; and
salts with inorganic or organic bases such as sodium ion, potassium ion, calcium ion, trimethyl ammonium and so forth.

The compounds represented by formula (I) or a salt thereof can be used as agrohorticultural pest control agents. The pests that can be controlled include various agricultural, forestry, horticultural, grain pests and sanitary pests that damage paddy rice, fruit trees, vegetables, other crops and flowering plants. In addition to insects, the above-mentioned pests include mites and nematodes, and the following pests are specifically recited.

As Lepidoptera (butterflies), for example, the following are recited: green cocklid (Parasa consocia), hibiscus looper (Anomis mesogona), swallowtail butterfly (Papilio xuthus), adzuki bean podworm (Matsumuraeses azukivora), adzuki bean borer (Ostrinia scapulalis), african armyworm (Spodoptera exempta), fall webworm (Hyphantria cunea), asian corn borer (Ostrinia furnacalis), oriental armyworm (Pseudaletia separata), casemaking clothes moth (Tinea translucens), mat rush worm (Bactra furfurana), rice skipper (Parnara guttata), rice leafroller (Marasmia exigua), ine-tsutomushi (Parnara guttata), pink stem borer (Sesamia inferens), sweetpotato leaf folder (Brachmia triannulella), oriental moth (Monema flavescens), cabbage looper (Trichoplusia ni), bean webworm (Pleuroptya ruralis), plum cankerworm (Cystidia couaggaria), long-tailed pea-blue (Lampides boeticus), coffee hawk moth (Cephonodes hylas), cotton bollworm (Helicoverpa armigera), oak caterpillar (Phalerodonta manleyi), giant bagworm (Eumeta japonica), large white (Pieris brassicae), tent caterpillar (Malacosoma neustria testacea), persimmon fruit moth (Stathmopoda masinissa), persimmon leafminer (Cuphodes diospyrosella), brown oak tortrix (Archips xylosteanus), turnip moth (Agrotis segetum), sugarcane shoot borer (Tetramoera schistaceana), common swallow tail (Papilio machaon hippocrates),

grape tree-borer (Endoclyta sinensis), apple leafminer (Lyonetia prunifoliella), apple leafminer (Phyllonorycter ringoneella), nut fruit tortrix (Cydia kurokoi), greenish chestnut moth (Eucoenogenes aestuosa), European grapevine Moth (Lobesia botrana), Chinese cochlid (Latoia sinica), persimmon bark borer (Euzophera batangensis), arrowhead moth (Phalonidia mesotypa), mulberry tiger moth (Spilosoma imparilis), mulberry pyralid (Glyphodes pyloalis), mulberry leaf roller (Olethreutes mori), common clothes moth (Tineola bisselliella), swift moth (Endoclyta excrescens), European grain moth (Nemapogon granellus), cherry tree borer (Synanthedon hector), codling moth (Cydia pomonella), diamondback moth (Plutella xylostella), rice leaffolder moth (Cnaphalocrocis medinalis), southern pinkborer (Sesamia calamistis), yellow stem borer (Scirpophaga incertulas), bluegrass webworm(Pediasia teterrellus), potato tuber moth(Phthorimaea operculella), lobster moth (Stauropus fagi persimilis), limabean pod borer (Etiella zinckenella), beet armyworm(Spodoptera exigua), swift moth (Palpifer sexnotata), lawn armyworm (Spodoptera mauritia), rice white stemborer (Scirpophaga innotata), spotted cutworm (Xestia c-nigrum), lawn grass cutworm (Spodoptera depravata), Mediterranean flour moth (Ephestia kuehniella), orange moth (Angerona prunaria), poplar prominent (Clostera anastomosis), soybean looper (Pseudoplusia includens), soybean pod worm (Matsumuraeses falcana), cape gooseberry budworm (Helicoverpa assulta), beet semi-looper (Autographa nigrisigna),
black cutworm (Agrotis ipsilon), tea tussock moth (Euproctis pseudoconspersa), summer fruit tortrix (Adoxophyes orana), tea leafroller (Caloptilia theivora), oriental tea tortrix (Homona magnanima), tobacco moth (Ephestia elutella), tea bagworm (Eumeta minuscula), scarce chocolate-tip (Clostera anachoreta), flax budworm (Heliothis maritima), grape leafroller (Sparganothis pilleriana), maize stalk borer (Busseola fusca), oriental tussock moth (Euproctis subflava), phytomimetic giant geometer (Biston robustum), tomato leaf miner (Tuta absoluta), corn earworm (Heliothis zea), eastern alchymist (Aedia leucomelas), pear stinging caterpillar (Narosoideus flavidorsalis), sorrel cutworm (Viminia rumicis), pear leaf miner (Bucculatrix pyrivorella), oriental fruit moth (Grapholita molesta), pear barkminer (Spulerina astaurota), pear fruit moth (Ectomyelois pyrivorella), rice stem borer (Chilo suppressalis), allium leafminer (Acrolepiopsis sapporensis), Indian-meal moth (Plodia interpunctella), cabbage webworm (Hellulla undalis), angoumois grain moth (Sitotroga cerealella), common cutworm (Spodoptera litura), tortrix spp. (Eucosma aporema), strawberry tortrix moth (Acleris comariana), persimmon cochlid (Scopelodes contractus), white-spotted tussock moth (Orgyia thyellina), fall armyworm (Spodoptera frugiperda), butterbur borer (Ostrinia zaguliaevi), rice green caterpillar (Naranga aenescens), tea cluster caterpillar (Andraca bipunctata), vine tree borer (Paranthrene regalis), grape horn worm (Acosmeryx castanea), grape leafminer (Phyllocnistis toparcha), grape berry moth (Endopiza viteana), European grape berry moth (Eupoecillia ambiguella), velvetbean caterpillar (Anticarsia gemmatalis), tobacco leaf worm (Cnephasia cinereipalpana), gypsy moth (Lymantria dispar), pine caterpillar (Dendrolimus spectabilis), soybean pod borer (Leguminivora glycinivorella), legume pod borer (Maruca testulalis), soybean pod worm (Matsumuraeses phaseoli),

soybean leafroller (Caloptilia soyella), citrus leafminer (Phyllocnistis citrella), mae-usuki-nomeiga (Omiodes indicatus), apple tortrix (Archips fuscocupreanus), three-spotted plusia (Acanthoplusia agnata), mulberry bagworm (Bambalina sp.), peach fruit moth (Carposina niponensis), yellow peach moth (Conogethes punctiferalis), peach Sesiidae (Synanthedon sp.), peach leafminer (Lyonetia clerkella), red helen (Papilio helenus), eastern pale clouded yellow (Colias erate poliographus), Japanese buff-tip moth (Phalera flavescens), common cabbageworm (Pieris rapae crucivora), Pieridae such as small white (Pieris rapae), brown tail moth (Euproctis similis), yam leafminer (Acrolepiopsis suzukiella), European corn borer (Ostrinia nubilalis), cabbage moth (Mamestra brassicae), Japanese giant looper (Ascotis selenaria), mugwort moth (Phtheochroides clandestina), leaf roller (Hoshinoa adumbratana), apple caterpillar (Odonestis pruni japonensis), apple dagger moth (Triaena intermedia), Adoxophyes orana fasciata (Adoxophyes orana fasciata), Grapholita inopinata (Grapholita inopinata), eyespotted bud moth (Spilonota ocellana), apple fruit licker (Spilonota lechriaspis), pear leaf worm (Illiberis pruni), apple fruit moth (Argyresthia conjugella), apple leaf miner (Caloptilia zachrysa), Asiatic leafroller (Archips breviplicanus), cotton looper (Anomis flava), pink bollworm (Pectinophora gossypiella), cotton leaf roller (Notarcha derogata), cotton caterpillar (Diaphania indica), tobacco budworm (Heliothis virescens), and cupreous bollworm (Earias cupreoviridis) and so forth.

As Hemiptera (stink bugs), for example, the following are recited: Nezara antennata (Nezara antennata), sorghum plant bug (Stenotus rubrovittatus), red-stripped stink bug (Graphosoma rubrolineatum), rice leaf bug (Trigonotylus coelestialium) and so forth, carrot bug (Aeschynteles maculatus), sweet potato yellow bug (Creontiades pallidifer), red cotton bug (Dysdercus cingulatus), circular black scale (Chrysomphalus ficus), California red scale (Aonidiella aurantii), large brown cicada (Graptopsaltria nigrofuscata), chinch bug (Blissusleucopterus), cottony cushion scale (Icerya purchasi), redbanded shield bug (Piezodorus hybneri), rice stink bug (Lagynotomus elongatus), orange headed leafhopper (Thaia subrufa), black rice bug (Scotinophara lurida), ibarahigenaga-aburamushi (Sitobion ibarae), shield bug (Stariodes iwasakii), coconut scale (Aspidiotus destructor), brokenbacked bug (Taylorilygus pallidulus), umekobu-aburamushi (Myzus mumecola), white prunicola scale (Pseudaulacaspis prunicola), pea aphid (Acyrthosiphon pisum), Coreid-bug (Anacanthocoris striicornis), Japanese garden fleahopper (Ectometopterus micantulus), ootogeshirahoshi-kamemushi (Eysarcoris lewisi), leaf-footed bug (Molipteryx fuliginosa), green leafhopper (Cicadella viridis), rice root aphid (Rhopalosophum rufiabdominalis), black scale (Saissetia oleae),

greenhouse whitefly (Trialeurodes vaporariorum), oak leafhopper (Aguriahana quercus), lygus bugs (Lygus spp.), European birch aphid (Euceraphis punctipennis), citrus scale (Andaspis kashicola), citricola scale (Coccus pseudomagnoliarum), oriental chinch bug (Cavelerius saccharivorus), chrysanthemum lace bug (Galeatus spinifrons), chrysanthemum aphid (Macrosiphoniella sanborni), yellow scale (Aonidiella citrina), brown malmorated stink bug (Halyomorpha mista), camphor lace bug (Stephanitis fasciicarina), camphor sucker (Trioza camphorae), rice bug (Leptocorisa chinensis), guercus sucker (Trioza quercicola), walnut lace bug (Uhlerites latius), grape leafhopper (Erythroneura comes), kuroashi-hosonaga-kamemushi (Paromius exiguus), camellia mining scale (Duplaspidiotus claviger), green rice leafhopper (Nephotettix nigropictus), oriental garden fleahopper (Halticiellus insularis), sugarcane leafhopper (Perkinsiella saccharicida), black apple sucker (Psylla malivorella), mulberry sucker (Anomomeura mori), comstock mealybug (Pseudococcus longispinis), mulberry scale (Pseudaulacaspis pentagona), cottony mulberry scale (Pulvinaria kuwacola), green leaf bug (Apolygus lucorum), seed bug (Togo hemipterus), tea aphid (Toxoptera aurantii), mealy bug (Saccharicoccus sacchari), sugarcane root aphid (Geoica lucifuga), pale sugarcane planthopper (Numata muiri), San Jose scale (Comstockaspis perniciosa), citrus snow scale (Unaspis citri),
foxglove aphid (Aulacorthum solani), shield bug (Eysarcoris ventralis), silver leaf whitefly (Bemisia argentifolii), Cicadella spectra (Cicadella spectra), oleander scale (Aspidiotus hederae), hyaline grass bug (Liorhyssus hyalinus), lacquer psylla (Calophya nigridorsalis), white backed planthopper (Sogatella furcifera), beam aphid (Megoura crassicauda), cabbage aphid (Brevicoryne brassicae), soybean aphid(Aphis glycines), rice bug (Leptocorisa oratorius), green rice leafhopper (Nephotettix virescens), taiwanhigenaga-aburamushi (Uroeucon formosanum), tobacco leaf bug (Cyrtopeltis tennuis), tobacco whitefly (Bemisia tabaci), European peach scale (Lecanium persicae), tea parlatoria scale (Parlatoria theae), white prunicola (Pseudaonidia paeoniae), tea green leafhopper (Empoasca onukii), brown-winged green bugs (Plautia stali), tulip bulb aphid (Dysaphis tulipae), potato aphid (Macrosiphum euphorbiae), azalea lace bug (Stephanitis pyrioides), Indian wax scale (Ceroplastes ceriferus), armored scale (Parlatoria camelliae), pale green plant bug (Apolygus spinolai), green rice leafhopper (Nephotettix cincticeps), polished green stink bug (Glaucias subpunctatus), mirid bug (Orthotylus flavosparsus), maize aphid (Rhopalosiphum maidis), maize planthopper (Peregrinus maidis), white spotted spined stink bug (Eysarcoris parvus), common bed bug (Cimex lectularius), abies psylla (Psylla abieti), brown rice planthopper (Nilaparvata lugens), tobira psylla (Psylla tobirae), cabbage bug (Eurydema rugosum), pear aphid (Schizaphis piricola), Psylla pyricola (Psylla pyricola), pear scale (Parlatoreopsis pyri), pear lace bug (Stephanitis nashi), Taxus mealybug (Dysmicoccus wistariae), pear scale (Lepholeucaspis japonica), root aphid (Sappaphis piri), turnip aphid (Lipaphis erysimi), onion aphid (Neotoxoptera formosana), hasu-kubire aburamushi (Rhopalosophum nymphaeae),

rose leafhopper (Edwardsiana rosae), fern scale (Pinnaspis aspidistrae), Plant louse (Psylla alni), a leafhopper (Speusotettix subfusculus), polyphagous leafhopper (Alnetoidia alneti), panicum planthopper (Sogatella panicicola), alfalfa plant bug (Adelphocoris lineolatus), small cotton bug (Dysdercus poecilus), black parlatoria scale (Parlatoria ziziphi), chestnut lace bug (Uhlerites debile), smaller brown planthopper (Laodelphax striatellus), painted bug (Eurydema pulchrum), slender rice bug (Cletus trigonus), spittle bug (Clovia punctata), Typhlocybine leafhopper (Empoasca sp.), brown soft scale (Coccus hesperidum), ground bug (Pachybrachius luridus), Japanese mealybug (Planococcus kraunhiae), timothy grass bug (Stenotus binotatus), grape leafhopper (Arboridia apicalis), aster leafhopper (Macrosteles fascifrons), sloe bug (Dolycoris baccarum), plant bug (Adelphocoris triannulatus), vine phylloxera (Viteus vitifolii), winter cherry bug (Acanthocoris sordidus), paddy bug (Leptocorisa acuta), bamboo chinch bug (Macropes obnubilus), rice stink bug (Cletus punctiger), rice stink bug (Riptortus clavatus), potato psyllid (Paratrioza cockerelli), willow froghopper (Aphrophora costalis), Japanese tarnished plant bug (Lygus disponsi), lygus bug (Lygus saundersi), pine mealybug (Crisicoccus pini), pine leafhopper (Empoasca abietis), matsumoto mealybug (Crisicoccus matsumotoi), cowpea aphid (Aphis craccivora), globular stink bug (Megacopta punctatissimum), Two-spotted sesame bug (Eysarcoris guttiger), purple scale (Lepidosaphes beckii),
citrus psylla (Diaphorina citri), brown citrus aphid (Toxoptera citricidus), citrus mealybug (Planococcus citri), citrus whitefly (Dialeurodes citri), citrous spiny whitefly (Aleurocanthus spiniferus), citrus mealybug (Pseudococcus citriculus), smaller citrus leafhopper (Zyginella citri), cottony citrus scale (Pulvinaria citricola), soft scale (Coccus discrepans), camphor scale(Pseudaonidia duplex), citrus scale (Pulvinaria aurantii), European fruit lecanium (Lecanium corni), southern green stink bug (Nezara viridula), wheat leaf bug (Stenodema calcaratum), bird-cherry aphid (Rhopalosiphum padi), corn leaf aphid (Sitobion akebiae), greenbug aphid (Schizaphis graminum), wheat leafhopper (Sorhoanus tritici), leafcurl plum aphid (Brachycaudus helichrysi), purple stink bug (Carpocoris purpureipennis), green peach aphid (Myzus persicae), mealy plum aphid (Hyalopterus pruni), willow aphid (Aphis farinose yanagicola), willow lace bug (Metasalis populi), arrowhead scale (Unaspis yanonensis), hibiscus psylla (Mesohomotoma camphorae), spirea aphid (Aphis spiraecola), apple aphid (Aphis pomi), oystershell scale (Lepidosaphes ulmi), black apple sucker (Psylla mali), apple leaf bug (Heterocordylus flavipes), apple leafcurling aphid (Myzus malisuctus), apple root aphid (Aphidonuguis mali), apple leafhopper (Orientus ishidai), apple aphid (Ovatus malicolens), woolly aphid (Eriosoma lanigerum), red wax scale (Ceroplastes rubens), and cotton aphid (Aphis gossypii) and so forth.

As Coleoptera (beetles), for example, the following are recited: monkeypod roundheaded borer (Xystrocera globosa), spider-lick (Paederus fuscipes), flower beetle (Eucetonia roelofsi), adzuki bean weevil (Callosobruchus chinensis), sweet potato weevils (Cylas formicarius), alfalfa weevil (Hypera postica), rice plant weevil (Echinocnemus squameus), rice leaf beetle (Oulema oryzae), rice rootworm (Donacia provosti), rice water weevil (Lissorhoptrus oryzophilus), sweetpotato leaf beetle (Colasposoma dauricum), West Indian sweetpotato weevil (Euscepes postfasciatus), Mexican bean beetle (Epilachna varivestis), bean weevil (Acanthoscelides obtectus), western corn rootworm (Diabrotica virgifera virgifera), plum borer (Involvulus cupreus), cucurbit leaf beetle (Aulacophora femoralis), pea weevil (Bruchus pisorum), leaf feeding ladybird (Epilachna vigintioctomaculata), corn sap beetle (Carpophilus dimidiatus), tortoise beetle (Cassida nebulosa), soybean flea beetle (Luperomorpha tunebrosa), striped flea beetle (Phyllotreta striolata), yellow-spotted longicorn beetle (Psacothea hilaris), yellow-dappled longicorn bettle (Aeolesthes chrysothrix), chestnut weevil (Curculio sikkimensis), dried fruit beetle (Carpophilus hemipterus), citrus flower chafer (Oxycetonia jucunda),

corn rootworms (Diabrotica spp.), scarab beetle (Mimela splendens), maize weevil (Sitophilus zeamais), red flour beetle (Tribolium castaneum), rice weevil (Sitophilus oryzae), depressed flour beetle (Palorus subdepressus), Japanese cockchafer (Melolontha japonica), white spotted longicorn beetle (Anoplophora malasiaca), yellow-dappled longicorn (Neatus picipes), Colorado potato beetle (Leptinotarsa decemlineata), southern corn rootworm (Diabrotica undecimpunctata howardi), hunting billbug (Sphenophorus venatus), tobacco beetle (Crioceris quatuordecimpunctata), Conotrachelus nenuphar (Conotrachelus nenuphar), chestnut weevil (Ceuthorhynchidius albosuturalis), brassica leaf beetle (Phaedon brassicae), daikon leaf beetle (Lasioderma serricorne), Japanese weevil (Sitona japonicus), brown chafer (Adoretus tenuimaculatus), yellow mealworm (Tenebrio molitor), leaf beetle (Basilepta balyi), lesser clover-leaf weevil (Hypera nigrirostris), brown-blackish beetle (Chaetocnema concinna), cupreous chafer (Anomala cuprea), yellowish elongate chafer (Heptophylla picea), spotted ladybird beetle (Epilachna vigintioctopunctata), northern corn rootworm (Diabrotica longicornis), flower beetle (Eucetonia pilifera), rootworms (Agriotes spp.), black carpet beetle (Attagenus unicolor japonicus), tropical legume leaf beetle (Pagria signata), soybean beetle (Anomala rufocuprea), smalleyed flour beetle (Palorus ratzeburgii), black fungus beetle (Alphitobius laevigatus), varied carpet beetle (Anthrenus verbasci), powderpost beetle (Lyctus brunneus), confused flour beetle (Tribolium confusum), two-striped leaf beetle (Medythia nigrobilineata), grape borer(Xylotrechus pyrrhoderus), potato leaf beetle (Epitrix cucumeris), Tomicus piniperda (Tomicus piniperda), Japanese pine sawyer (Monochamus alternatus), Japanese beetle (Popillia japonica), bean blister beetle (Epicauta gorhami), maize weevil (Sitophilus zeamais), peach curculio (Rhynchites heros), vegetable weevil (Listroderes costirostris), cowpea bruchid (Callosobruchus maculatus), common leaf weevil (Phyllobius armatus), apple blossom weevil (Anthonomus pomorum), alder chrysomelid beetle (Linaeidea aenea), and boll weevil (Anthonomus grandis) and so forth.

As Dipteran (flies), for example, the following are recited: common house mosquito (Culex pipiens pallens), spinach leafminer (Pegomya hyoscyami), pea leafminer (Liriomyza huidobrensis), house fly (Musca domestica), rice stem maggot (Chlorops oryzae), rice whorl maggot (Hydrellia sasakii), Japanese rice leaf miner (Agromyza oryzae), rice leaf miner (Hydrellia griseola), rice leaf miner (Hydrellia griseola), Ophiomyia phaseoli (Ophiomyia phaseoli), melon fly (Dacus cucurbitae), cherry fly (Drosophila suzukii), Japanese cherry fruit fly (Rhacochlaena japonica), false stable fly (Muscina stabulans), Phoridae such asphorid fly (Megaselia spiracularis), Clogmia albipunctata (Clogmia albipunctata), rice crane fly(Tipula aino), black blow fly(Phormia regina), house mosquito(Culex tritaeniorhynchus), rice crane malaria vector (Anopheles sinensis), cabbage maggot (Hylemya brassicae), soybean pod gall midge (Asphondylia sp.), bean seed fly (Delia platura), onion fly (Delia antiqua), cherry fruit fly (Rhagoletis cerasi), autogenic house mosquito(Culex pipiens molestus Forskal), Mediterranean fruit fly (Ceratitis capitata), sciarid fly (Bradysia agrestis), beet leaf miner (Pegomya cunicularia), vegetable leafminer (Liriomyza sativae), celery miner fly (Liriomyza bryoniae), pea leaf miner (Chromatomyia horticola), stone leek leafminer (Liriomyza chinensis), tropical house mosquito (Culex quinquefasciatus), yellow fever mosquito (Aedes aegypti), Asian tiger mosquito (Aedes albopictus), American serpentine leafminer (Liriomyza trifolii), vegetable leafminer (Liriomyza sativae), oriental fruit fly (Dacus dorsalis), citrus fruit fly (Dacus tsuneonis), orange wheat blossom midge (Sitodiplosis mosellana), wheat stem maggot (Meromuza nigriventris), Mexican fruit fly (Anastrepha ludens), and apple maggot (Rhagoletis pomonella) and so forth.

As Hymenoptera (bees), for example, the following are recited: parthenogenetic ant (Pristomyrmex pungens), wingless wasp, pharaoh ant (Monomorium pharaohnis), oozu ant (Pheidole noda), turnip sawfly (Athalia rosae), oriental chestnut gall wasp (Dryocosmus kuriphilus) and so forth, negro ant (Formica fusca japonica), giant hornets, buff-tailed bumblebee (Athalia infumata), large rose sawfly (Arge pagana), cabbage sawfly (Athalia japonica), leaf-cutter ant (Acromyrmex spp. ), fire ant (Solenopsis spp.), apple argid sawfly (Arge mali) and black house ant (Ochetellus glaber) and so forth.

As Orthoptera (hoppers), for example, the following are recited: northern rice katydid (Homorocoryphus lineosus), mole crickets (Gryllotalpa sp.), short-homed grasshoppers (Oxya hyla intricata), rice grasshopper (Oxya yezoensis), migratory locust (Locusta migratoria), lesser paddy grasshopper (Oxya japonica), northern rice katydid (Homorocoryphus jezoensis), and emma field cricket (Teleogryllus emma) and so forth.

As Thysanoptera, for example, the following are recited: redbanded thrips (Selenothrips rubrocinctus), rice thrips (Stenchaetothrips biformis), rice aculeated thrips (Haplothrips aculeatus), Japanese gall-forming thrips (Ponticulothrips diospyrosi) and so forth, honeysuckle thrips (Thrips flavus), grass thrips (Anaphothrips obscurus), camphor thrips (Liothrips floridensis), gladiolus thrips (Thrips simplex), chrysanthemum thrips (Thrips nigropilosus), greenhouse thrips (Heliothrips haemorrhoidalis), mulberry thrips (Pseudodendrothrips mori), composite thrips (Microcephalothrips abdominalis), castanopis gall thrips (Leeuwenia pasanii), castanopis thrips (Litotetothrips pasaniae), citrus thrips (Scirtothrips citri), Chinese thrips (Haplothrips chinensis), oriental soybean thrips (Mycterothrips glycines), Japanese flower thrips (Thrips setosus), yellow tea thrips (Scirtothrips dorsalis), black tea thrips (Dendrothrips minowai), red clover thrips (Haplothrips niger), onion thrips (Thrips tabaci), black onion thrips (Thrips alliorum), Hawaiian flower thrips (Thrips hawaiiensis), predatory thrips (Haplothrips kurdjumovi), cocksfoot thrips (Chirothrips manicatus), flower thrips(Frankliniella intonsa), loquat thrips (Thrips coloratus), Western flower thrips (Franklinella occidentalis), melon thrips (Thrips palmi), yellow lily thrips (Frankliniella lilivora) and lily thrips (Liothrips vaneeckei) and so forth.

As acari, for example, the following are recited: ticks such as scrub typhus mite (Leptotrombidium akamushi), bean spidermite (Tetranychus ludeni), American dog tick (Dermacentor variabilis), spider mite (Tetranychus truncatus), tropical rat mite (Ornithonyssus bacoti), dog acne mite (Demodex canis), and so forth, hawthorn spider mite (Tetranychus viennensis), kanzawa spidermite (Tetranychus kanzawai), brown dog tick (Rhipicephalus sanguineus) and so forth; a Cheyletus mite (Cheyletus malaccensis), mould mite (Tyrophagus putrescentiae), house dust mite (Dermatophagoides farinae), redback spider (Latrodectus hasseltii), Taiwanese Dermacentor tick (Dermacentor taiwanicus), pink tea rust mite (Acaphylla theavagrans), broad mite (Polyphagotarsonemus latus), tomato russet mite (Aculops lycopersici), northern fowl mite (Ornithonyssus sylviarum), two spotted spidermite (Tetranychus urticae), Japanese pear rust mite (Eriophyes chibaensis), sarcoptic mange mite (Sarcoptes scabiei), bush tick (Haemaphysalis longicornis), blacklegged tick (Ixodes scapularis), spinach mold mite (Tyrophagus similis), thin cheyletid (Cheyletus eruditus), citrus redmite (Panonychus citri), southern cheyletid (Cheyletus moorei), flat mite (Brevipalpus phoenicis), ear mite (Octodectes cynotis), an epidermoptid (Dermatophagoides ptrenyssnus), Japanese blood tick (Haemaphysalis flava), Ixodes ovatus tick (Ixodes ovatus), eriophyid rust mite (Phyllocoptruta citri), apple rust mite (Aculus schlechtendali), European red mite (Panonychus ulmi), lone star tick (Amblyomma americanum), parasitoid mite (Dermanyssus gallinae), bulb mite (Rhyzoglyphus robini), and bee mites (Sancassania sp.) and so forth.

As Isopteran, for example, the following are recited: amami termite (Reticulitermes miyatakei), western dry wood termite (Incisitermes minor), Formosan subterranean termite (Coptotermes formosanus), damp-wood termite (Hodotermopsis japonica), kanmon termite (Reticulitermes sp. ), yellowlegged termite (Reticulitermes flaviceps amamianus), kushimototermite (Glyptotermes kushimensis), Japanese dampwood termite (Coptotermes guangzhoensis), koshu termite (Neotermes koshunensis), kodama termite (Glyptotermes kodamai), satsuma termite (Glyptotermes satsumensis), Drywood termite (Cryptotermes domesticus), black-winged subterranean termite (Odontotermes formosanus), nakajima termite (Glyptotermes nakajimai), soil feeder (Pericapritermes nitobei), and Japanese termite (Reticulitermes speratus) and so forth.

As blattodea, for example, the following are recited: smokybrown cockroach (Periplaneta fuliginosa), German cockroach (Blattella germanica), oriental cockroach (Blatta orientalis), brown cockroach (Periplaneta brunnea), false German cockroach (Blattella lituricollis), Japanese cockroach (Periplaneta japonica), and American cockroach (Periplaneta americana) and so forth.

As nematodes, for example, the following are recited: strawberry bud nematode (Nothotylenchus acris), rice white tip nematode (Aphelenchoides besseyi), nematode (Pratylenchus penetrans), cotton root-knot nematode (Meloidogyne hapla), a root-knot nematode (Meloidogyne incognita), yellow potato cyst nematode (Globodera rostochiensis), a root-knot nematode (Meloidogyne javanica), soybean cyst nematode (Heterodera glycines), dry rot nematode (Pratylenchus coffeae), wheat root-knot nematode (Pratylenchus neglectus), citrus nematode (Tylenchus semipenetrans).

As mollusca, for example, channeled applesnail (Pomacea canaliculata), giant African land snail (Achatina fulica), slug (Meghimatium bilineatum), cha-koora slug (Lehmannina valentiana), yellow slug (Limax flavus), and Korean round snail (Acusta despecta sieboldiana) and so forth are recited.

The agrohorticultural pest control agent containing the present inventive compound represented by the general formula (I) or a salt thereof as an active ingredient has a marked controlling effect on the above-exemplified pests, which are injurious to paddy field crops, upland crops, fruit trees, vegetables and other crops, flowers and ornament plants, and so forth so that the desired effect of the present inventive agrohorticultural pest control agent can be achieved by applying the insecticide to nursery facilities, paddy fields, upland fields, fruit trees, vegetables, other crops or flowers and ornament plants before the emergence of the pests or when their emergence has been confirmed. In addition, an application form using the so-called systemic effect is available, whereby the present inventive compound is absorbed from the roots through or not through the soil by being treated to the nursery soil of crops ornamental plants and so forth; the picking-in hole soil at a transplantation; the plant roots; the plan foot; or the cultural water of a water culture.

The useful plants to which the present inventive agrohorticultural pest control agent can be applied are not particularly limited, and the following plants can be recited to as examples thereof:

cereals such as rice, barley, wheat, rye, oat, corn and so forth; beans and peas such as soybean, red bean, broad bean, pea, kidney-bean, peanut and so forth; fruit trees such as apple, citrus trees and fruits, pear, grape, peach, plum, cherry, walnut, chestnut, almond, banana) and so forth; leafy and fruit vegetables such as cabbage, tomato, spinach, broccoli, lettuce, onion, stone-leek (chive, stone-leek), Spanish paprika, eggplant, strawberry, okra, pepper, chinese chive and so forth; root crops such as carrot, potato, sweet potato, taro, radish, lotus rhizome, turnip, burdock, garlic, scallion and so forth; processing crops such as cotton, flax, beet, hop, sugar can, sugar beet, olive, gum, coffee, tobacco, tea and so forth; cucurbitaceous plants such as pumpkin, cucumber, musk melon, water melon, melon and so forth; pasture plants such as orchard grass, sorghum, timothy, clover, alfalfa and so forth; lawn grasses such as mascarenegrass, bent grass and so forth; perfumery crops such as lavender, rosemary, thyme, parsley, pepper, ginger and so forth; flowers and ornamental plants such as chrysanthemum, rose, carnation, orchid, tulip, lily and so forth; garden-trees such as ginkgo tree, cherry tree, gold-leaf plant and so forth; and timber woods such as white fir, silver fir, pine, hatchet-leaved arbor-vitae, Japan cedar, Japanese eypress, eukalyptus and so forth.

The aforementioned useful plants also include those to which resistance to HPPD inhibitors such as isoxaflutole, ALS inhibitors such as imazethapyr or thifensulfuron-methyl, EPSP synthetase inhibitors such as glyphosate, glutamine synthetase inhibitors such as glufosinate, acetyl-CoA carboxylase inhibitors such as sethoxydim and herbicides such as bromoxynil, dicamba, 2,4-D and so forth has been conferred by a classical breeding method or genetic engineering technique.

Examples of the useful plant to which resistance has been conferred by a classical breeding method include rape, wheat, sunflower and rice resistant to imidazolinone ALS inhibitory herbicides such as imazethapyr, which are already commercially available under a product name of Clearfield (registered trademark). Likewise there is soy bean to which resistance to sulfonylurea ALS inhibitory herbicides such as thifensulfuron-methyl has been conferred by a classical breeding method, which is already commercially available under a product name of STS soy bean. Likewise examples to which resistance to acetyl-CoA carboxylase inhibitors such as trione oxime or aryloxy phenoxypropionic acid herbicides has been conferred by a classical breeding method include SR corn.

The plant to which resistance to acetyl-CoA carboxylase inhibitors has been conferred is described in Proceedings of the National Academy of Sciences of the United States of America (Proc. Natl. Acad. Sci. USA), vol. 87, pp. 7175-7179 (1990) and so forth. A variation of acetyl-CoA carboxylase resistant to an acetyl-CoA carboxylase inhibitor is reported in Weed Science, vol. 53, pp. 728-746 (2005) and so forth and a plant resistant to acetyl- CoA carboxylase inhibitors can be generated by introducing a gene of such an acetyl-CoA carboxylase variation into a plant by genetically

engineering technology or by introducing a variation conferring resistance into a plant acetyl-CoA carboxylase; furthermore, plants resistant to acetyl-CoA carboxylase inhibitors or ALS inhibitors or the like can be generated by introducing a site-directed amino acid substitution variation into an acetyl-CoA carboxylase gene or the ALS gene of the plant by introduction a nucleic acid into which has been introduced a base substitution variation represented Chimeraplasty Technique (Gura T. 1999. Repairing the Genome's Spelling Mistakes. Science 285: 316-318) into a plant cell. The present inventive agrohorticultural pest control agent can be used for these plants too.

Furthermore, examples of toxins expressed in such genetically engineered crops include: insecticidal proteins derived from Bacillus cereus or Bacillus popilliae; δ-endotoxins such as CrylAb, CrylAc, CrylF, CrylFa2, Cry2Ab, Cry3A, Cry3Bbl or Cry9C derived from Bacillus thuringiensis; insecticidal proteins such as VIPl, VIP2, VIP3 or VIP3A; insecticidal proteins derived from nematodes; toxins generated by animals such as scorpion toxin, spider toxin, bee toxin or insect-specific neurotoxins; mold fungi toxins; plant lectin; agglutinin; protease inhibitors such as a trypsin inhibitor, a serine protease inhibitor, patatin, cystatin or a papain inhibitor; ribosome- inactivating proteins (RIP) such as lycine, corn-RIP, abrin, luffin, saporin or briodin; steroid- metabolizing enzymes such as 3-hydroxysteroid oxidase, ecdysteroid-UDP-glucosyl transferase or cholesterol oxidase; an ecdysone inhibitor; HMG-CoA reductase; ion channel inhibitors such as a sodium channel inhibitor or calcium channel inhibitor; juvenile hormone esterase; a diuretic hormone receptor; stilbene synthase; bibenzyl synthase; chitinase; and glucanase and so forth.

Moreover, toxins expressed in such genetically engineered crops also include: hybrid toxins, partially deleted toxins and modified toxins of δ-endotoxin proteins such as CrylAb, CrylAc, CrylF, CrylFa2, Cry2Ab, Cry3A, Cry3Bbl, Cry9C, Cry34Ab or Cry35Ab and insecticidal proteins such as VIPl, VIP2, VIP3 or VIP3A. The hybrid toxins are produced from a new combination of the different domains of such proteins, using a genetic engineering technique. As the partially deleted toxins, CrylAb comprising a deletion of a portion of an amino acid sequence is known. In the modified toxins one or more amino acids of natural toxins are substituted.

Examples of such toxins and genetically engineered plants capable of synthesizing such toxins are described in EP0 374 753, WO 93/07278, WO 95/34656, EP0 427 529, EP451 878, WO 03/052073 and so forth.

The toxins contained in these genetically engineered plants confer resistance to plants in particular to coleopteran pests, hemipteran pests, dipteran pests, lepidopteran pests or nematodes. The present inventive agrohorticultural pest control agent can be used in combination or in system with such technique.

In order to control various pests, the present inventive agrohorticultural pest control agent is applied to the plants on which emergence of the pests or nematodes is expected, either as it is or in the form of a dilution or suspension in a proper quantity of water or the like at a dosage effective for the control of the pests or nematodes. For instance, with the aim of controlling the appearance of pests or nematodes on fruit trees, cereals and vegetables, the composition may be directly used for foliage treatment or the composition may also be used for seed treatments such as immersion of seeds in the agent solution, seed coating, calper treatment or the like or absorption from the root by soil treatment or the like, such as incorporation into total soil layer, row treatment, soil incorporation, cell seedling treatment, prickling-in-hole treatment, plant foot treatment, top dressing, nursery box application of rice, submerged application and so forth. In addition, application of t the present inventive agrohorticultural pest control agent to the nutrient solution in the water culture, the use by fumigation, and the injection into tree stalks and so forth are also usable.

Furthermore, the present inventive agrohorticultural pest control agent may be applied to the plants on which emergence of the pests or nematodes is expected, either as it is or in the form of a dilution or suspension in a proper quantity of water or the like at a dosage effective for the control of the pests or nematodes; for example, it may be used, in addition to being sprayed to stored grain pests, house pests, sanitary pests, forest pests and so forth, by being pasted onto building materials or as a fumigant or bait and so forth.

As the method of treating seeds, a method of dipping seeds in a diluted or undiluted liquid preparation of the liquid or solid composition and thereby making the agent permeate into the seeds; a method of mixing a solid or liquid preparation with seeds for the sake of powder coating and thereby making the agent adhere to the seed surface; a method of mixing the preparation with an adhesive carrier such as resin, polymer or the like and coating seeds with such an adhesive mixture; a method of spraying the preparation to the neighborhood of seeds simultaneously with planting and so forth can be referred to.

"Seed" to be treated with the seed treatment means a plant body of the initial stage of cultivation used for reproduction of plants, encompassing not only the seeds but also plant bodies for nutrient reproduction such as bulb, tuber, seed tuber, stock bud, aerial tuber, scaly bulb or stalks for cuttage and so forth.

"Soil" or "cultivation carrier" for plants in carrying out the using method of the present invention means a support for the cultivation of a plant, in particular, a support in which the roots grow; their material quality is not limited, any material being acceptable as far as the plant can grow therein. For example, so-called soils, nursery mat, water and so forth can be used, specific examples for the material being sand, pumice, vermiculite, diatomaceous earth, agar, gelatinous materials, polymeric materials, rock wool, glass wool, wood chips, bark and so forth.

As the method for spraying onto foliage parts of crops or stored grain pests, sanitary pests, forest pests and so forth, spraying a liquid formulation such as an emulsifiable concentrate, flowable agent and so forth or a solid formulation

such as a wettable powder or wettable granule and so forth having properly been diluted with water, spraying a dust, or fumigation and so forth can be referred to.

As the method of soil application, applying a liquid preparation either diluted with water or undiluted onto the plant foot, nursery bed for raising seedlings or the like, spraying granules onto the plant foot or nursery bed, spraying a dust, a wettable powder, a wettable granule or granules onto the soil and mixing with the whole soil either before seeding or before transplantation, spraying a dust, wettable powder, wettable granule, granules or the like onto planting holes, planting rows and so forth can be recited.

As the method for applying to a nursery box of paddy field rice, even though the preparation form may be varied depending on the time of application such as application at the sowing period, greening period or transplanting period, applying in the form of a dust, wettable granule, granules and so forth can be recited. Application by mixing with the soil is also possible, which application is mixing with soil and a dust, wettable granule or granules, examples thereof being mixing into the bed soil, covering soil or whole soil. Further possible method is application by merely making the soil and various formulations into layers.

For applying to a paddy field, usually to a paddy field in a submerged state, a solid preparation such as a jumbo-pack, granules, wettable granules and so forth or a liquid formulation such as a flowable, emulsifiable concentrate and so forth are sprinkled. Otherwise, it is also possible to sprinkle or inject an appropriate agent as it is or in the form of a mixture with fertilizers into the soil at the time of transplantation. Further possible is applying chemical solution of an emulsifiable concentrate to the water inlet or water flow source of the irrigating system, whereby a labor-saving application is achieved with water supplied.

In case of upland field crops, application to the cultivation carrier surrounding the seeds or plant bodies in the period from the seeding to the seedling raising is available. For plants where seeds are directly sown to the field, in addition to direct application to seeds, application onto the base of hills during the cultivation period is preferable. Sprinkling granules or irrigating with a liquid formulation after dilution with water or without dilution and so forth are possible. Another preferable treatment is to mix granules with cultivation carriers before seeding and to sow seeds thereafter.

In cases where cultured plants to be transplanted are treated at the seeding time or in the seedling raising period, in addition to direct treatment onto seeds, irrigating treatment onto a seedling raising bed with a liquefied form or to sprinkling granules are preferable. Further, applying granules to the planting holes at the time of set-planting or mixing into the cultivation carrier in the neighborhood of the sites of transplantation are also preferable treatment.

The present inventive agrohorticultural pest control agent is in general used after having been formulated into a form which is convenient in use by the standard method for formulating agrochemicals.

Namely, the present inventive derivative represented by the general formula I or a salt thereof may be used after having been blended optionally together with an adjuvant in a proper proportion and prepared into a suitable preparation form such as suspension, emulsifiable concentrate, soluble concentrate, wettable powder, wettable granules, granules, dust tablets or packs through dissolution, separation, suspension, mixing, impregnation, adsorption or sticking.

The composition of the present invention, i.e. agrohorticultural pest control agent or animal parasite controlling agent, may contain in addition to the active ingredient additive components which are commonly used for agricultural formulations or animal parasite controlling agents if necessary. As such additive components, a carrier such as a solid carrier or a liquid carrier, a surfactant, a dispersant, a wetting agent, a binder, an adhesion-imparting agent, a thickener, a coloring agent, an extender, a spreader, an anti-freezing agent, an anti-caking agent, a disintegrating agent and a stabilizing agent and so forth can be recited. Furthermore, an antiseptic agent, plant pieces may be used as additive components if necessary. These additive components may be used either alone or in combination of two or more.

As the solid carrier, for example, a natural mineral such as quartz, clay, kaolinite, pyrophillite, sericite, talc, bentonite, acid clay, attapulgite, zeolite or diatomaceous earth; an inorganic salt such as calcium carbonate, ammonium sulfate, sodium sulfate or potassium chloride, synthetic silicic acid or synthetic silicate; an organic solid carrier such as starch, cellulose or plant powder (saw dust, coconut shellflower, corncob and tobacco stem); a plastic carrier such as polyethylene, polypropylene or polyvinylidene chloride; or urea, inorganic hollow materials, plastic hollow materials or fumed silica (white carbon) can be recited. These may be used either alone or in combination of two or more.

As the liquid carrier, for example, alcohols such as a monohydric alcohol such as methanol, ethanol, propanol, isopropanol or butanol or a polyhydric alcohol such as ethylene glycol, diethylene glycol, propylene glycol, hexylene glycol, polyethylene glycol, polypropylene glycol or glycerol; a polyhydric alcohol derivative such as propylene type glycol ether; a ketone such as acetone, methyl ethyl ketone, methyl isobutyl ketone, diisobutyl ketone or cyclohexanone; an ether such as ethyl ether, dioxane, ethylene glycol monoethyl ether, dipropyl ether or tetrahydrofuran; an aliphatic hydrocarbon such as normal paraffin, naphthene, isoparaffin, kerosine or mineral oil; an aromatic hydrocarbon such as benzene, toluene, xylene, solvent naphtha or alkyl naphthalene; a halogenated hydrocarbon such as dichloroethane, chloroform or carbon tetrachloride; an ester such as ethyl acetate, diisopropyl phthalate, dibutyl phthalate, dioctyl phthalate or dimethyl adipate; a lactone such as $\gamma$-butyrolactone; an amide such as dimethylformamide, diethylformamide, dimethylacetamide or N-alkylpyrrolidinone; a nitrile such as acetonitrile; a sulfur compound such as dimethylsulfoxide;

a vegetable oil such as soybean oil, rapeseed oil, cotton oil or castor oil; or water can be recited. These may be used either alone or in combination of two or more.

As surfactants used as a dispersant or wetting agent, the following can be recited: a nonionic surfactant such as a sorbitan fatty acid ester, a polyoxyethylene sorbitan fatty acid eater, a sucrose fatty acid ester, a polyoxyethylene fatty acid ester, a polyoxyethylene resin acid ester, a polyoxyethylene fatty acid diester, a polyoxyethylene alkyl ether, a polyoxyethylene alkyl phenyl ether, a polyoxyethylene dialkyl phenyl ether, a polyoxyethylene alkyl phenyl ether formalin condensate, a polyoxyethylene polyoxypropylene block copolymer, an alkyl polyoxyethylene polypropylene block polymer ether, a polyoxyethylene alkylamine, a polyoxyethylene fatty acid amide, a polyoxyethylene fatty acid bisphenyl ether, a polyalkylene benzyl phenyl ether, a polyoxyalkylene styryl phenyl ether, an acetylenediol, a polyoxyalkylene-added acetylenediol, a polyoxyethylene ether type silicon, an ester type silicon, a fluorinated surfactant, a polyoxyethylene castor oil or a polyoxyethylene hardened castor oil; an anionic surfactant such as an alkyl sulfate, a polyoxyethylene alkyl ether sulfate, a polyoxyethylene alkyl phenyl ether sulfate, a polyoxyethylene styryl phenyl ether sulfate, an alkyl benzenesulfonate, a lignin sulfonate, an alkylsulfosuccinate, a naphthalenesulfonate, an alkylnaphthalenesulfonate, a salt of a formalin condensate of naphthalenesulfonate, a salt of a formalin condensate of an alkylnaphthalenesulfonate, a fatty acid salt, a polycarboxylic acid salt, an N-methyl-fatty acid sarcosinate, a resin acid salt, a polyoxyethylene alkyl ether phosphate or a polyoxyethylene alkyl phenyl ether phosphate; a cationic surfactant such as a laurylamine hydrochloride, a stearylamine hydrochloride, an oleylamine hydrochloride, a stearylamine acetate, a stearylaminopropylamine acetate, an alkyltrimethylammonium chloride or an alkyldimethylbenzalkonium chloride; or an amphoteric surfactant such as an amino acid type or a betain type. These surfactants may be used either alone or in combination of two or more.

As the binder or adhesion-imparting agent, for example, carboxymethylcellulose or its salt, dextrin, water-soluble starch, xanthan gum, guar gum, sucrose, polyvinylpyrrolidone, gum arabic, polyvinyl alcohol, polyvinyl acetate, polysodium acrylate, a polyethylene glycol having an average molecular weight of 6,000 to 20,000, a polyethylene oxide having an average molecular weight of 100,000 to 5,000,000, phospholipids (e.g. cephalin, lecithin, etc.), cellulose powders, dextrins, processed starches, polyaminocarboxylic acid chelate compounds, cross-linked polyvinylpyrrolidone, copolymers of maleic acid and styrenes, (meth)acrylic acid copolymers, half esters of polymers consisting of polyhydric alcohols and dicarboxylic anhydrides, water-soluble salts of polystyrene sulphonic acid, paraffins, terpenes, polyamide resins, polyacrylates, polyoxyethylene, waxes, polyvinylalkyl ethers, alkylphenol formalin condensates, synthetic resin emulsions and so forth can be recited.

As the thickener, for example, a water-soluble polymer such as xanthan gum, guar gum, diutan gum, carboxylmethylcellulose, polyvinylpyrrolidone, carboxyvinyl polymer, an acrylic polymer, a starch compound or polysaccharide; or an inorganic fine powder such as high purity bentonite or fumed silica (white carbon) can be recited.

As the coloring agent, for example, an inorganic pigment such as iron oxide, titanium oxide or Prussian blue; or an organic dye such as an arizarin dye, an azo dye or a metal phthalocyanine dye can be recited.

As the anti-freezing agent, for example, a polyhydric alcohol such as ethylene glycol, diethylene glycol, propylene glycol or glycerol can be recited.

As the additive component for an anti-caking agent or disintegrating agent, for example, starch, alginic acid, a polysaccharide such as mannose or galactose, polyvinylpyrrolidone, fumed silica (white carbon), ester gum or petroleum resin, sodium tripolyphosphate, sodium hexamethaphosphate, stearic acid metal salt, a cellulose powder, dextrin, a methacrylate copolymer, a polyvinylpyrrolidone, a polyaminocarboxylic acid chelate compound, a styrene sulfonate/isobutylene/maleic anhydride copolymer or a starch/polyacrylonitrile graft copolymer can be recited.

As the stabilizer, for example, a drying agent such as zeolite, quick lime or magnesium oxide; an anti-oxidation agent such as a phenol type, an amine type, a sulfur type or a phosphorus type; or an ultraviolet absorber such as a salicylic acid type or a benzophenone type can be recited.

As the antiseptic, for example, potassium sorbate or 1,2-benzthiazolin-3-one can be recited.

In addition, if necessary, a functional spreader, an activity enhancer such as piperonyl butoxide, an anti-freezing agent such as propylene glycol, an antioxidant such as BHT or other additive agents such as an UV absorber can be used.

The content of the active ingredient compound may be varied according to the need; the content can properly be selected from the range between 0.01 and 90 parts by mass in terms of 100 parts by mass of the present inventive agrohorticultural pest control agent. For example, for dusts, granules, emulsifiable concentrates or wettable powders, the suitable content is from 0.01 to 50% parts by mass, i.e. 0.01 to 50% parts by mass for the entire mass of the agrohorticultural pest control agent.

The applying dosage of the harmful organism controlling agent of the present invention varies depending upon various factors such as, for example, a purpose, insect pests to be controlled, a growth state of a plant, tendency of insect pest emergence, weather, environmental conditions, a preparation form, an application method, an application site and an application time; the dosage of the active ingredient compound may be properly chosen in a range of 0.001 g to 10 kg, preferably 0.01 g to 1 kg per 10 ares depending upon the purpose.

The present inventive agrohorticultural pest control agent can be used in admixture with other agrohorticultural pest control agents, acaricides, nematicides, fungicides or biological pesticides, in order to expand both the spectrum

of controllable diseases and pests, extend the controlling period or reduce the dosage; in addition, use in admixture with herbicides, plant growth regulators, fertilizer and so forth depending on the use occasions.

As other agrohorticultural pest control agents, acaricides, nematicides to be used for such an aim, the following are, for example, recited:

3,5-xylyl methylcarbamate (XMC), fenobucarb (BPMC), Bt toxin insecticidal compound, CPCBS (chlorfenson), DCIP (dichlorodiisopropyl ether), D-D (1, 3-Dichloropropene), DDT, NAC, O-4-dimethylsulfamoylphenyl O,O-diethyl phosphorothioate (DSP), O-ethyl O-4-nitrophenyl phenylphosphonothioate (EPN), tripropylisocyanurate (TPIC), acrinathrin, azadirachtin, acynonapyr, azinphos-methyl, acequinocyl, acetamiprid, acetoprole, acephate, abamectin, afidopyropen, avermectin avermectin-B, amidoflumet, amitraz, alanycarb, aldicarb, aldoxycarb, aldrin, alpha-endosulfan, alpha-cypermethrin, albendazole, allethrin, isazofos, isamidofos, isoamidofos, isoxathion, isocycloseram, isofenphos, isoprocarb (MIPC), epsilon-metofluthrin, epsilon-momfluorothrin, ivermectin, imicyafos, imidac1oprid, imiprothrin, indazapyoxamet, indoxacarb, esfenvalerate, ethiofencarb, ethion, ethiprole, etoxazole, ethofenprox, ethoprophos, etrimfos, emamectin, emamectin-benzoate, endosulfan, empenthrin, oxazosulfyl, oxamyl, oxydemeton-methyl, oxydeprofos (ESP), oxibendazole, oxfendazole, potassium oleate, sodium oleate, cadusafos, kappa-bifenthrin, cartap, carbary1, carbosulfan, carbofuryl, gamma-cyhalothrin,
xylylcarb, quinalphos, kinoprene, chinomethionat, cloethocarb, clothianidin, clofentezine, chromafenozide, chlorantraniliprole, chlorethoxyfos, chlordimeform, chlordane, chlorpyrifos, chlorpyrifos-methyl, chlorphenapyr, chlorfenson, ch1orfenvinphos, chlorfluazuron, chlorobenzilate, chlorobenzoate, chloroprallethrin, Kelthane (dicofol), salithion, cyhalodiamide, cyanophos (CYAP), diafenthiuron, diamidafos, cyantraniliprole, theta-cypermethrin, cyetpyrafen, dienochlor, cyenopyrafen, dioxabenzofos, diofenolan, sigma-cypermethrin, cyclaniliprole,
dichlofenthion (ECP), cyclobutrifluram, cycloprothrin, dichlorvos (DDVP), dicloromezotiaz, disulfoton, dinotefuran, cyhalodiamide, cyhalothrin, cyphenothrin, cyfluthrin, diflubenzuron, cyflumetofen, cyproflanilide, diflovidazin, cyhexatin, cypermethrin, dimethylvinphos, dimethoate, dimpropyridaz, dimefluthrin, silafluofen, cyromazine, spidoxamat, spinetoram, spinosad, spirodiclofen, spirotetramat, spirobudifen, spiropidion, spiromesifen, sulfluramid, sulprofos, sulfoxaflor, zeta-cypermethrin, diazinon, tau-fluvalinate, dazomet, thiacloprid, thiamethoxam, tioxazafen,
thiodicarb, thiocyclam, thiosultap, thiosultap-sodium, thionazin, thiometon, tiorantraniliprole, deet, dieldrin, tetrachlorantraniliprole, tyclopyrazoflor, tetrach1orvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, doramectin, tralopyril, tralomethrin, transfluthrin, triazamate, triazuron, trichlamide, trichlorphon: DEP, trifluenfuronate, triflumezopyrium, triflumuron, tolfenpyrad, naled: BRP, nicofluprole, nithiazine, nitenpyram, novaluron, noviflumuron, hydroprene, vaniliprole, vamidothion,
parathion, parathion-methyl, halfenprox, halofenozide, bistrifluron, bisultap, hydramethylnon, hydroxy propyl starch, binapacryl, pyflubumide, bifenazate, bifenthrin, pymetrozine, pyraclorfos, pyrafluprole, pyridafenthion, pyridaben, pyridalyl, pyrifluquinazon, pyriprole, pyriproxyfen, pirimicarb, pyrimidifen, pyriminostrobin, pirimiphos-methy1, pyrethrins, fiproni1, fenazaquin, fenamiphos, bromopropylate, fenitrothion (MEP), fenoxycarb, fenothiocarb, phenothrin, fenobucarb, fensulfothion, fenthion (MPP), phenthoate (PAP), fenvalerate, fenpyroximate, fenpropathrin, fenbendazole, fenmezoditiaz, fosthiazate, formetanate, butathiofos, buprofezin, furathiocarb, prallethrin, fluacrypyrim, fluazaindolizine, fluazinam, fluazuron, fluensulfone, fluxametamide,
fluchlordiniliprole, flucycloxuron, flucythrinate, fluvalinate, flupyradifurone, flufiprole, flupyradifurone, flupyrazofos, flupyrimin, flupyroxystrobin, flufenerim, flufenoxystrobin, flufenoxuron, flufenzine, flufenoprox, fluproxyfen, flubrocythrinate, fluhexafon, flubendiamide, flupentiofenox, flumethrin, flurimfen, prothiofos, protrifenbute, flonicamid, propaphos, propargite:BPPS, profenofos, broflanilide, profluthrin, propoxur:PHC, flometoquin, $\alpha$-alpha-bromadiolone, bromopropylate, beta-cyfluthrin,
hexaflumuron, hexythiazox, heptafluthrin, heptenophos, permethrin, benclothiaz, bendiocarb, benzpyrimoxan, bensu1tap, benzoximate, benfuracarb, phoxim, phosalone, fosthiazate, fosthietan, phosphamidon, phosphocarb, phosmet: PMP, polynactins, formetanate, formothion, phorate, machine oil, malathion, milbemycin, milbemycin-A, milbemectin, mecarbam, mesulfenfos, methomyl, metaldehyde, metaflumizone, methamidophos, metam-ammonium, metam-sodium, methiocarb,
methidathion: DMTP, methylisothiocyanate, methylneodecanamide, methylparathion, metoxadiazone, methoxychlor, methoxyfenozide, metofluthrin, methoprene, metolcarb, meperfluthrin, mevinphos, monocrotophos, monosultap, momfluorothrin, lambda-cyhalothrin, ryanodine, lufenuron, rescalure, resmethrin, lepimectin, rotenone, levamisol hydrochloride, fenbutatin oxide, morantel tartarate, methyl bromide, cyhexatin, calcium cyanamide, calcium polysulfide, sulfur, and nicotine-sulfate, or a salt thereof.

As compounds having fungicidal activity, the following can be recited for example: aureofungin, azaconazole, azithiram, acypetacs, acibenzolar, acibenzolar-S-methyl, azoxystrobin, anilazine, amisulbrom, aminopyrifen, ampropylfos, ametoctradin, allyl alcohol, aldimorph, amobam, isotianil, isovaledione, isopyrazam, isofetamid, isoflucypram, isoprothiolane, ipconazole, ipfentrifluconazole, ipflufenoquin, iprodione, iprovalicarb, iprobenfos, imazalil, iminoctadine, metam,

iminoctadine-albesilate, iminoctadine-triacetate, imibenconazole, inpyrfluxam, uniconazole, uniconazole-P, echlomezole, edifenphos, etaconazole, ethaboxam, ethirimol, etem, ethoxyquin, etridiazole, enestroburin, enoxastrobin, epoxiconazole, oxadixyl, oxathiapiprolin, oxycarboxin, copper-8-quinolinolate, oxytetracycline, copper-oxinate, oxpoconazole, oxpoconazole-fumarate, oxolinic acid, octhilinone, ofurace, orysastrobin, metam-sodium, kasugamycin, carbamorph, carpropamid, carbendazim,

carboxin, carvone, quinazamid, quinacetol, quinoxyfen, quinofumelin, chinomethionat, quinomethionate, captafol, captan, kiralaxyl, quinconazole, quintozene, guazatine, cufraneb, cuprobam, coumoxystrobin, glyodin, griseofulvin, climbazole, cresol, kresoxim-methyl, chlozolinate, clotrimazole, chlobenthiazone, chloraniformethan,

chloranil, chlorquinox, chloropicrin, chlorfenazole, chloroinconazide, chlorodinitronaphthalene, chlorothalonil, chloroneb, salicylanilide, zarilamid, cyazofamid, diethyl pyrocarbonate, diethofencarb, cyclafuramid, diclocymet, dichlozoline, diclobutrazol, dichlofluanid, cycloheximide, dichlobentiazox, diclomezine,

dicloran, dichlorophen, dichlone, disulfiram, ditalimfos, dithianon, diniconazole, diniconazole-M, zineb, dinocap, dinocton, dinosulfon, dinoterbon, dinobuton, dinopenton, dipymetitrone, dipyrithione, diphenylamine, difenoconazole, cyflufenamid, diflumetorim, cyproconazole, cyprodinil, cyprofuram, cypendazole, simeconazole, dimethirimol, dimethomorph, cymoxanil, dimoxystrobin, ziram, silthiofam, streptomycin, spiroxamine, sultropen, sedaxane, seboctylamine,

zoxamide, dazomet, thiadiazin, tiadinil, thiadifluor, thiabendazole, tioxymid, thioquinox, thiochlorfenphim, thiophanate, thiophanate-methyl, thifluzamide, thicyofen, thioquinox, thiram, decafentin, tecnazene, tecloftalam, tecoram, tetraconazole, debacarb, dehydroacetic acid, tebuconazole, tebufloquin, dodicin, dodine, Dodecylbenzenesulphonic acid bisethylenediamine copper [II] salt (DBEDC), dodemorph,

drazoxolon, triadimenol, triadimefon, triazbutil, triazoxide, triamiphos, triarimol, trichlamide, triclopyricarb, tricyclazole, triticonazole, tridemorph, tributyltin oxide, triflumizole, trifloxystrobin, triforine, tolylfluanid, tolclofos-methyl, tolprocarb, natamycin, nabam, nitrostyrene, nitrothal-isopropyl, nuarimol, copper nonylphenol sulfonate, halacrinate, validamycin, valifenalate, harpin protein, picarbutrazox, bixafen, picoxystrobin, picobenzamide, pydiflumetofen, bithionol, bitertanol, hydroxyisoxazole, hydroisoxazole-potassium, binapacryl, biphenyl, piperalin, hymexazol, pyraoxystrobin, pyracarbolid,

pyraclostrobin, pyraziflumid, pyrazophos, pyrapropoyne, pyrametostrobin, pyriofenone, pyridinitril, pyrifenox, pydiflumetofen, pyrisoxazole, pyridachlometyl, pyrifenox, pyribencarb, pyriminostrobin, pyrimethanil, pyroxychlor, pyroxyfur, pyroquilon, vinclozolin, ferbam, famoxadone, fenapanil, fenamidone, fenaminosulf, fenaminstrobin, fenarimol, fenitropan, fenoxanil, ferimzone, ferbam, fentin, fenpiclonil, fenpicoxamid, fenpyrazamine, fenbuconazole, fenfuram, fenpropidin, fenpropimorph, fenhexamid,

phthalide, buthiobate, butylamine, bupirimate, fuberidazole, Sblasticidin-S, furametpyr, furalaxyl, fluacrypyrim, fluazinam, fluindapyr, fluoxastrobin, fluoxapiprolin, fluoxytioconazole, fluotrimazole, fluopicolide, fluopimomide, fluopyram, fluoroimide, furcarbanil, fluxapyroxad, fluquinconazole, furconazole, furconazole-cis, fludioxonil, flusilazole, flusulfamide, flutianil, flutolanil,

flutriafol, flufenoxadiazam, flufenoxystrobin, furfural, flubeneteram, furmecyclox, flumetylsulforim, flumetover, flumorph, proquinazid, prochloraz, procymidone, prothiocarb, prothioconazole, pronitridine, propamocarb, propiconazole, propineb, furophanate, probenazole, bromuconazole, florylpicoxamid, hexachlorobutadiene, hexaconazole, hexylthiofos, bethoxazin, benalaxyl, benalaxyl-M, benodanil, benomyl, pefurazoate, benquinox,

penconazole, benzamorf, pencycuron, benzohydroxamic acid, benzovindiflupyr, bentaluron, benthiazole, benthiavalicarb, benthiavalicarb-isopropyl, penthiopyrad, penflufen, boscalid, phosdiphen, fosetyl, fosetyl-Al, polyoxins, polyoxorim, polycarbamate, folpet, formaldehyde, machine oil, maneb, mancozeb, mandipropamid, mandestrobin, myclozolin, myclobutanil, mildiomycin, milneb, mecarbinzid, methasulfocarb, metazoxolon, metam, metam-sodium, metalaxyl,

metalaxyl-M, metarylpicoxamid, metiram, methyl isothiocyanate, mepthyldinocap, Metyltetraprole, metconazole, metsulfovax, methfuroxam, metominostrobin, metrafenone, mepanipyrim, mefenoxam, mefentrifluconazole, meptyldinocap, mepronil, mebenil, iodomethane, rabenzazole, methyl bromide,

inorganic fungicides such as benzalkonium chloride, basic copper chloride, basic copper sulfate and silver, sodium hypochlorote, cupric hydroxide, wettable sulfur, calcium polysulfide, potassium hydrogen carbonate, sodium hydrogen carbonate, sulfur, copper sulfate anhydride, nickel dimethyldithiocarbamate, copper compounds such as 8-oxine, zinc sulfate, and copper sulfate pentahydrate, or a salt thereof.

As compounds having herbicidal activity, the following can be recited for example: 1-naphthylacetamide, 2,4-PA, 2,3,6-TBA, 2,4,5-T, 2,4,5-TB, 2,4-D, 2,4-DB, 2,4-DEB, 2,4-DEP, 3,4-DA, 3,4-DB, 3,4-DP, 4-CPA, 4-CPB, 4-CPP, MCP, MCPA, MCPA-thioethyl, MCPB, ioxynil, aclonifen, azafenidin, acifluorfen, aziprotryne, azimsulfuron, asulam, acetochlor, atrazine, atraton,

anisuron, anilofos, aviglycine, abscisic acid, amicarbazone, amidosulfuron, amitrole, aminocyclopyrachlor, aminopyralid, amibuzin, amiprophos-methyl, ametridione, ametryn, alachlor, allidochlor, alloxydim, alorac, iofensulfuron, isouron, isocarbamid, isoxachlortole, isoxapyrifop, isoxaflutole, isoxaben, isocil, isonoruron, isoproturon, isopropalin, isopolinate, isomethiozin, inabenfide, ipazine, iptriazopyrid, ipfencarbazone, iprymidam, imazaquin, imazapic, imazapyr, imazamethapyr, imazamethabenz, imazamethabenz-methyl, imazamox, imazethapyr, imazosulfuron, indaziflam, indanofan, indolebutyric acid, uniconazole-P, eglinazine, esprocarb, ethametsulfuron, ethametsulfuron-methyl, ethalfluralin, ethiolate, ethychlozate ethyl, ethidimuron, etinofen, ethephon, ethoxysulfuron, ethoxyfen, etnipromid, ethofumesate, etobenzanid, epyrifenacil, epronaz, erbon, endothal, oxadiazon, oxadiargyl, oxaziclomefone, oxasulfuron, oxapyrazon, oxyfluorfen, oryzalin, orthosulfamuron, orbencarb, cafenstrole, cambendichlor, carbasulam, carfentrazone, carfentrazone-ethyl, karbutilate, carbetamide, carboxazole, quizalofop, quizalofop-P, quizalofop-ethyl, xylachlor, quinoclamine, quinonamid, quinclorac, quinmerac, cumyluron, clacyfos, cliodinate, glyphosate, glufosinate, glufosinate-P, credazine, clethodim, cloxyfonac, clodinafop, clodinafop-propargyl, chlorotoluron, clopyralid, cloproxydim, cloprop, chlorbromuron, clofop, clomazone, chlomethoxyni 1, chlomethoxyfen, clomeprop, chlorazifop, chlorazine, cloransulam, chloranocryl, chloramben, cloransulam, cloransulam-methyl, chloridazon, chlorimuron, chlorimuron-ethyl, chlorsulfuron, chlorthal, chlorthiamid, chlortoluron, chlornitrofen, chlorfenac, chlorfenprop, chlorbufam, chlorphthalim, chlorflurazole, chlorflurenol, chlorprocarb, chlorpropham, chlormequat, chloreturon, chloroxynil, chloroxuron, chlorotoluron, chloropon, cypyrafluone, saflufenacil, cyanazine, cyanatryn, di-allate, diuron, diethamquat, dioxopyritrione, dicamba, cycluron, cycloate, cycloxydim, diclosulam, cyclosulfamuron, cyclopyranil, cyclopyrimorate, dichlorprop, dichlorprop-P, dichlobenil, dichlobenz-methyl, diclofop, diclofop-methyl, dichlormate, dichloralurea, diquat, cisanilide, disul, siduron, dithiopyr, dinitramine, cinidon-ethyl, dinosam, cinosulfuron, dinoseb, dinoterb, dinofenate, dinoprop, cyhalofop-butyl, diphenamid, difenoxuron, difenopenten, difenzoquat, cybutryne, cyprazine, cyprazole, diflufenican, diflufenzopyr, dipropetryn, cypromid, cyperquat, gibberellin, simazine, dimexano, dimesulfazet, dimethachlor, dimidazon, dimethametryn, dimethenamid, simetryn, simeton, dimepiperate, dimefuron, cinmethylin, swep, sulglycapin, sulcotrione, sulfallate, sulfentrazone, sulfosulfuron, sulfometuron, sulfometuron-methyl, secbumeton, sethoxydim, sebuthylazine, terbacil, daimuron, dazomet, dalapon, thiazafluron, thiazopyr, tiafenacil, thiencarbazone, thiencarbazone-methyl, tiocarbazil, tioclorim, thiobencarb, thidiazimin, thidiazuron, thifensulfuron, thifensulfuron-methyl, desmedipham, desmetryn, tetflupyrolimet, tetrafluron, thenylchlor, tebutam, tebuthiuron, terbumeton, tepraloxydim, tefuryltrione, tembotrione, delachlor, terbacil, terbucarb, terbuchlor, terbuthylazine, terbutryn, topramezone, tralkoxydim, triaziflam, triasulfuron, triafamone, tri-allate, trietazine, tricamba, triclopyr, tridiphane, tritac, tritosulfuron, tripyrasulfone, trifludimoxazin, triflusulfuron, triflusulfuron-methyl, trifluralin, trifloxysulfuron, tripropindan, tribenuron, tribenuron-methyl, tribenuron, trifop, trifopsime, trimeturon, tolpyralate, naptalam, naproanilide, napropamide, nicosulfuron, nitralin, nitrofen, nitrofluorfen, nipyraclofen, neburon, norflurazon, noruron, barban, paclobutrazol, paraquat, parafluron, haloxydine, halauxifen, haloxyfop, haloxyfop-P, haloxyfop-methyl, halosafen, halosulfuron, halosulfuron-methyl, bilanafos, bixlozone, picloram, picolinafen, bicyclopyrone, bispyribac, bispyribac-sodium, pydanon, pinoxaden, bipyrazone, bifenox, piperophos, hymexazol, pyraclonil, pyrasulfotole, pyrazoxyfen, pyrazosulfuron, pyrazosulfuron-ethyl, pyrazolate, bilanafos, pyraflufen-ethyl, pyriclor, pyridafol, pyrithiobac, pyrithiobac-sodium, pyridate, pyriftalid, pyributicarb, pyriflubenzoxim, pyribenzoxim, pyrimisulfan, primisulfuron, pyriminobac-methyl, pyroxasulfone, pyroxsulam, fenasulam, phenisopham, fenuron, fenoxasulfone, fenoxaprop, fenoxaprop-P, fenoxaprop-ethyl, phenothio1, fenoprop, phenobenzuron, fenquinotrione, fenthiaprop, fenteracol, fentrazamide, phenmedipham, phenmedipham-ethyl, fenpyrazone, butachlor, butafenacil, butamifos, buthiuron, buthidazole, butylate, buturon, butenachlor, butroxydim, butralin, flazasulfuron, flamprop, furyloxyfen, prynachlor, primisulfuron-methyl, fluazifop, fluazifop-P, fluazifop-butyl, fluazolate, flucloraminopry, flucloraminopry-tefuryl, fluroxypyr, fluothiuron, fluometuron, fluoroglycofen, flurochloridone, fluorodifen, fluoronitrofen, fluoromidine, flucarbazone, flucarbazone-sodium, fluchloralin, flusulfinam, flucetosulfuron, fluthiacet, fluthiacet-methyl, flupyrsulfuron, flufenacet, flufenican, flufenoximacil, flufenpyr, flupropacil, flupropanate, flupoxam, flumioxazin, flumiclorac, flumiclorac-pentyl, flumipropyn, flumezin, fluometuron, flumetsulam, fluridone, flurtamone, fluroxypyr, pretilachlor, proxan, proglinazine, procyazine, prodiamine, prosulfalin, prosulfuron, prosulfocarb, propaquizafop, propachlor, propazine, propanil, propyzamide, propisochlor, prohydrojasmon, propyrisulfuron, propham, profluazol, profluralin, prohexadione-calcium, propoxycarbazone, propoxycarbazone-sodium,

profoxydim, bromacil, brompyrazon, prometryn, prometon, bromoxynil, bromofenoxim, bromobutide, bromobonil, florasulam, florpyrauxifen, hexachloroacetone, hexazinone, pethoxamid, benazolin, penoxsulam, pebulate, beflubutamid, beflubutamid-M, vernolate, perfluidone,
bencarbazone, benquitrione, benzadox, benzipram, benzylaminopurine, benzthiazuron, benzfendizone, bensulide, bensulfuron-methyl, benzoylprop, benzobicyclon, benzofenap, benzofluor, bentazone, pentanochlor, benthiocarb, pendimethalin, pentoxazone, benfluralin, benfuresate, fosamine,
fomesafen, foramsulfuron, forchlorfenuron, maleic hydrazide, mecoprop, mecoprop-P, medinoterb, mesosulfuron, mesosulfuron-methyl, mesotrione, mesoprazine, methoprotryne, metazachlor, methazole, metazosulfuron, methabenzthiazuron, metamitron, metamifop, metam, methalpropalin, methiuron, methiozolin, methiobencarb, methyldymron, metoxuron, metosulam, metsulfuron, metsu1furon-methy1, metflurazon, metobromuron, metobenzuron, methometon, metolachlor, metribuzin, mepiquat-chloride, mefenacet,
mefluidide, monalide, monisouron, monuron, monochloroacetic acid, monolinuron, molinate, morfamquat, iodosulfuron, iodosulfuron-methyl-sodium, iodobonil, iodomethane, lactofen, lancotrione, rimisoxafen, linuron, rimsulfuron, lenacil, rhodethanil, calcium peroxide, methyl bromide, or a salt thereof.

Examples of biopesticide include, without being limited thereto, *Agrobacterium radiobacter* (e.g., Galltrol-A (trademark registered) manufactured by AgBioChem, CA using strain K84, Nogall (trademark registered) manufactured by Becker Underwood, US using strain K1026), *Agrobacterium radiobacter* (e.g., Bacterose (trademark registered) manufactured by NIHON NOHYAKU Co., Ltd. using strain 84), *Ampelomyces quisqualis* (e.g., AQ 10 (trademark registered) manufactured by IntrachemBio Italia & Co. KG using strain AQ10), *Aspergillus flavus* (e.g., Afla-Guard (trademark registered) manufactured by Syngenta, AF36 (trademark registered) manufactured by Arizona Cotton Research and Protection Council, US using strain AF36), *Aureobasidium pullulans* (e.g., Botector (trademark registered), a mixture of blastospores of strain DSM14940, blastospores of strain DSM14941, manufactured by bio-ferm, GmbH),

*Bacillus amyloliquefaciens* (e.g., Impression Clear (trademark registered) manufactured by Idemitsu Agri using strain AT-332, Avogreen (trademark registered) manufactured by University of Pretoria using strain B246, Bacstar (trademark registered) manufactured by Etec Crop Solutions, NZ using strain D747, Shelter (trademark registered) manufactured by Dagutat Bio lab, ZA using strain DB101, Artemis (trademark registered) manufactured by Dagutat Bio lab, ZA using strain DB102, RhizoVital (trademark registered) manufactured by ABiTEP, DE using strain FZB42, Kodiak (trademark registered) manufactured by Bayer CropScience AG, DE using strain GB03, Subtilex (trademark registered) manufactured by Becker Underwood, US using strain MBI600, Amplitude using strain F727 manufactured by Marrone Bio Innovations, Inc.), *Bacillus cepacia* (e.g., Deny Stine (trademark registered) manufactured by Microbial Products), *Bacillus cereus* (e.g., Mepichlor (trademark registered) manufactured by Arysta, US using strain BP01), *Bacillusfirmus* (e.g., BioNeem (trademark registered) manufactured by AgoGreen using strain 1-1582),
*Bacillus lacticola* (e.g., a product manufactured by Micro Flo Company), *Bacillus lactimorbus* (e.g., a product manufactured by Micro Flo Company), *Bacillus lactis* (e.g., a product manufactured by Micro Flo Company), *Bacillus laterosporus* (e.g., Bio-Tode (trademark registered) manufactured by Agro-Organics, SA), *Bacillus licheniformis* (e.g., EcoGuard Biofungicide (trademark registered) manufactured by Novozymes using strain SB3086), *Bacillus maroccanus* (e.g., a product manufactured by Micro Flo Company), *Bacillus megaterium* (e.g., Bioarc (trademark registered) manufactured by Bio Arc using strain YFM3.25), *Bacillus metiens* (e.g., a product manufactured by Micro Flo Company), *Bacillus mojavensis* (e.g., a product manufactured by Probelte, Sa using strain SR11), *Bacillus mycoides* (e.g., BmJ (trademark registered) manufactured by Certis USA using isolate J.),
*Bacillus nigrificans* (e.g., a product manufactured by Micro Flo Company), *Bacillus popilliae* (e.g., Cronox (trademark registered) manufactured by Bio Crop, CO), *Bacillus pumilus* (e.g., Integral F-33 (trademark registered) manufactured by Becker Underwood, US using strain BU F-33, Yield Shield (trademark registered) manufactured by Bayer CropScience AG, DE using strain GB34, Sonata (trademark registered) manufactured by Bayer CropScience LP, US using strain QST2808), *Bacillus simplex* (e.g., Momi-Hope WP (trademark registered) manufactured by Arysta LifeScience Corporation using strain CGF2856), *Bacillus sphaericus* (e.g., VectoLex (trademark registered) manufactured by Valent BioSciences, US using serotype H5a5b strain 2362),
*Bacillus subtilis* (e.g., Botokiller WP (trademark registered) manufactured by Idemitsu Agri Co., Taegro (trademark registered) manufactured by Novozyme Biologicals, Inc. US using strain FZB24, SERENADE MAX (trademark registered) manufactured by Bayer CropScience LP, US using strain QST713/AQ713, Serenade-DPZ (trademark registered) using strain AQ30002, EcoShot (trademark registered) manufactured by KUMIAI CHEMICAL INDUSTRY Co., Ltd. using strain D747, Agrocare WP (trademark registered) manufactured by Nisso Green Co., Ltd. using strain HAI-0404, Botopika WP (trademark registered) manufactured by Idemitsu Kosan Co., Ltd. using strain MBI600, BaciStar WP (trademark registered) manufactured by Arysta LifeScience Corporation using strain Y1336, Companion Biological Fungicide Wettable Powder using strain GB03 manufactured by Growth Products Ltd), *Bacillus thuringiensis* (e.g., VectoBac (trademark registered) manufactured by Valent BioSciences, US using strain AM65-52),

*Bacillus thuringienses aizawai* (e.g., XenTari (trademark registered) manufactured by Bayer CropScience AG, DE using strain ABTS-1857, Florbac WG (trademark registered) manufactured by Valent BioSciences, US using serotype H-7, Agree WG Biological Insecticide using strain GC-91 manufactured by Certis USA), *Bacillus thuringiensis* subspecies. *Aegypti* (e.g., Agerin (trademark registered)), *Bacillus thuringienses israelensis* (e.g., Aquabac (trademark registered) manufactured by Becker Microbial Products IL using strain BMP 144),

*Bacillus thuringienses kurstaki* (e.g., a product manufactured by Becker Microbial Products, IL using strain BMP 123, Dipel ES (trademark registered) manufactured by Valent BioSciences, US using strain HD, BMP 123 using strain BMP123 manufactured by Becker Microbial Products, BMP144/Aquabac using strain BMP144 manufactured by Becker Microbial Products, Dipel 10G using strain ABTS-351 manufactured by Valent U. S.A. LLC., Condor Wettable Powder using strain EG2348 manufactured by Certis US, Crymax Bioinsecticide using strain EG7841 manufactured by Certis USA, Deliver Biological Insecticide using strain SA-12 manufactured by Certis USA, Bioprotec PLUS using strain EVB-113-19 manufactured by AEF Global), Bacillus thuringiensis galleria (e.g., beetleGONE! Manufactured by Phyllom BioProducts or boreGONE! Using strain SDS-502 manufactured by Phyllom BioProducts), *Bacillus thuringiensis* var. *colmeri* (e.g., TianBaoBTc (trademark registered) manufactured by Changzhou Jianghai Chemical Factory), *Bacillus thuringienses tenebrionis* (e.g., Novodor FC (trademark registered) manufactured by BioFa DE using strain NB 176), *Bacillus thuringiensis* var. *san diego* (e.g., M-One (trademark registered) from Bacillus thuringiensis var. san diego), *Beauveria bassiana* (e.g., Naturalis (trademark registered) manufactured by Intrachem Bio Italia, Bove Max (trademark registered) manufactured by Novozymes using strain CG716, BioLisa Madara (trademark registered) manufactured by Idemitsu Kosan Co., Ltd. using strain F-263, BotaniGard WP (trademark registered) manufactured by ARYSTA using strain GHA, balEnce using strain HF23 manufactured by Terragena, Inc., BotaniGard ES using strain GHA manufactured by BioWorks Inc., BioCeres WP using strain ANT-03manufactured by BioSafe Systems),

*Beauveria brongniartii* (e.g., Beaupro (trademark registered) manufactured by Andermatt Biocontrol AG), *Bradyrhizobium japonicum* (e.g., Optimize (trademark registered) manufactured by Novozymes), *Burkholderia* spp. (e.g., MBI-206 TGAI (trademark registered) manufactured by Marrone Bio Innovations using strain A396), *Candida oleophila* (e.g., Aspire manufactured by Ecogen Inc., US using strain 1-82, Nexy manufactured by BioNext using strain O), *Candida saitoana* (e.g., BIOCURE (trademark registered) manufactured by Micro Flo Company, US (BASF SE)), *Chaetomium cupreum* (e.g., BIOKUPRUM (trademark registered) manufactured by AgriLife), *Chaetomium globosum* (e.g., Rivadiom (trademark registered) manufactured by Rivale), *Chromobacterium subtsugae* (e.g., Grandevo (trademark registered) manufactured by Marrone Bio Innovations using strain PRAA4-1T),

*Cladosporium cladosporioides* (e.g., from Cladosporium cladosporioides), *Clonostachys rosea* f. *catenulate* (e.g., PRESTOP (trademark registered) manufactured by Verdera, Finland using strain J1446), *Colletotrichum gloeosporioides* (e.g., Collego (trademark registered) manufactured by Agriultural Research Initiatives), *Coniothyrium minitans* (e.g., Contans (trademark registered) manufactured by Encore Technologies, LLC using strain CON/M/91-08), *Cryptococcus albidus* (e.g., YieldPlus (trademark registered) manufactured by Anchor Bio Technologies, ZA), *Delftia acidovorans* (e.g., BioBoost (trademark registered) manufactured by Brett Young Seeds using strain RAY209), *Dilophosphora alopecuri* (e.g., Twist Fungus (trademark registered)), *Drechsrela monoceras* (e.g., Tasumato herbicide (trademark registered) manufactured by Mitsui Chemicals Agro, Inc. using strain MTB-951), *Entomophthora virulenta* (e.g., Vektor (trademark registered) manufactured by Ecomic), *Fusarium oxysporum* (e.g., Maruka light manufactured by Eisai Seikaken, Inc. using strain 101-2), *Fusarium oxysporum* (e.g., Fusaclean (trademark registered) manufactured by Natural Plant Protection using strain Fo47),

*Gliocladium* spp. (e.g., Prestop (trademark registered) manufactured by AgBio Inc. using strain J1446, a product manufactured by W. F. Stoneman Company LLC using strain 321U), *Hirsutella thompsonii* (e.g., Mycohit (trademark registered) manufactured by Agro Bio tech Research Centre, IN), *Lactobacillus acidophilus* (e.g., Fruitsan (trademark registered) manufactured by Inagrosa Industrias Agrobiologicas, S.A.), *Lactobacillus plantarum* (e.g., Lactoguard WP manufactured by Meiji Seika Pharma Co., Ltd. using strain BY), *Lecanicillium lecanii* (e.g., Mycotal (trademark registered) manufactured by Koppert/Arysta using conidiospores of strain KV01), *Metarhizium anisopliae* (e.g., BIO 1020 (trademark registered) manufactured by Bayer CropScience using strain F52, Pirates G (trademark registered) manufactured by Arysta LifeScience Corporation using strain SMZ-2000, Bio-Blast using strain ESC1 manufactured by LidoChem Inc),

*Metarhizium anisopliae var. acridum* (e.g., GreenMuscle (trademark registered) manufactured by Biological Control Products, GreenGuard (trademark registered) manufactured by Becker Underwood, US), *Metschnikowia fructicola* (e.g., Shemer (trademark registered) manufactured by Bayer CropScience), *Microdochium dimerum* (e.g., ANTIBOT (trademark registered) manufactured by Agrauxine, France), *Microsphaeropsis ochracea* (e.g., Microx (trademark registered) manufactured by Prophyta), *Monacrosporium phymatopagum* (e.g., Nemahiton (trademark registered) manufactured by Tomoe Kagaku Kogyo K.K.), *Mucor haemelis* (e.g., BioAvard (trademark registered) manufactured by Indore Biotech Inputs & Research), Muscodor albus (e.g., Arabesque using strain QST 20799 manufactured by Bayer Crop Science), *Myrothecium verrucaria* (e.g., DiTera (trademark registered) manufactured by Valent Biosciences using strain AARC-0255), *Paecilomyces fumosoroseus* (e.g., PreFeRal (trademark registered) WG manufactured by Biobest using strain apopka 97, Preferred WP manufactured by Tokai Bussan Co. Ltd., No Fly (trademark registered) manufactured by Natural Industries Inc. (Novozymes company) using strain FE 9901), *Paecilomyces lilacinus* (e.g., BioAct WG (trademark registered) manufactured by Prophyta using strain 251),

*Pacilimyces tenuipes* (e.g., Gottu A manufactured by Idemitsu Kosan Co., Ltd. using strain T1), *Paecilomyces variotii* (e.g., Nemaquim (trademark registered) manufactured by Quimia, MX using strain Q-09), *Paenibacillus polymyxa* (e.g., Topseed (trademark registered) manufactured by Green Biotech Company Ltd. using strain AC-1), *Paenibacillus poppiliae* (e.g., Milky spore disease (trademark registered) manufactured by St. Gabriel Laboratories), *Pasteuria nishizawae* (e.g., oyacyst LF/ST (trademark registered) manufactured by Pasteuria Bioscience or Clariva pn using strain Pn1 manufactured by Syngenta), *Pasteuria penetrans* (e.g., Pasteuria (trademark registered) manufactured by Pasteuria Bioscience), *Pasteuria usagae* (e.g., Econem (trademark registered) manufactured by Pasteuria Bioscience),

Pantoea agglomerans (e.g., Bloomtime Biological FD Biopesticide using strain E325 manufactured by Nufarm US), *Pectobacterium carotovorum* (e.g., Biokeeper (trademark registered) manufactured by Nissan Chemical Corporation, EcoMate (trademark registered) manufactured by KUMIAI CHEMICAL INDUSTRY Co., Ltd. using CGE234M403), *Phoma macrostroma* (e.g., Phoma H (trademark registered) manufactured by Scotts, US using strain 94-44B), *Penicillium bilaii* (e.g., Jump Start (trademark registered) manufactured by Novozymes), *Phlebiopsis gigantea* (e.g., ROTSOP (trademark registered) manufactured by Verdera, Finland using strain FOC PG B22/SP1190/3.2), *Pochonia chlamydosporia* var. *catenulata* (e.g., KlamiC (trademark registered) manufactured by The National Center of Animal and Plant Health (CENSA); CU), *Pseudomonas aureofaciens* (e.g., Spot-Less Biofungicide (trademark registered) manufactured by Eco Soils Systems, CAusing strain TX-1), *Pseudomonas chlororaphis* (e.g., ATEze (trademark registered) manufactured by EcoSoil Systems using strain 63-28, Cedomon (trademark registered) manufactured by Bioagri, S. using strain MA 342), *Pseudomonas fluorescens* (e.g., Frostban D (trademark registered) manufactured by Frost Technology Corp. using strain 1629RS, Blightban (trademark registered) manufactured by Blightban using strain A506, Konae Fukudo (trademark registered) manufactured by Taki Chemical Co., Ltd. using strain FPT-9601, Cell Nae Genki (trademark registered), a mixture of strains FPT-9601, FPH-9601, manufactured by Taki Chemical Co., Ltd., Vegikeeper (trademark registered) manufactured by Arysta LifeScience Corporation using strain G7090), *Pseudomonas proradix* (e.g., Proradix (trademark registered) manufactured by Sourcon Padena),

*Pseudomonas resinovorans* (e.g., Solanacure (trademark registered) manufactured by Agricultural Research Council, SA), *Pseudomonas syringae* (e.g., Biosave (trademark registered) manufactured by EcoScience, US using strain MA-4, Frostban C (trademark registered) manufactured by Frost Technology Corp using strain 742RS, Bio-save 10LP Biological Fungicide using strain ESC10 manufactured by Jet Harvest Systems, Bio-Save 11 LP Biological Fungicide using strain ESC11 manufactured by Jet Harvest Systems), *Pseudomonas* spp. (e.g., Masterpiece WP (trademark registered) manufactured by Nippon Soda Co., Ltd. using strain HAI-0804, Momi-Genki WP manufactured by Nissan Chemical Corporation using strain CAB-02), *Pseudozyma aphidis* (e.g., a product manufactured by Yissum Research Development Company of the Hebrew University of Jerusalem), *Pseudozyma flocculosa* (e.g., Sporodex L manufactured by Plant Products Co. Ltd., CA using strain PF-A22 UL), *Pythium oligandrum* (e.g., Polyversum (trademark registered) manufactured by Bioprepraty, CZ using strain DV74 or strain M1), *Reynoutria sachlinensis* (e.g., REGALIA (trademark registered) manufactured by Marrone BioInnovations, US),

*Rhizopogon amylopogon* (e.g., Myco-Sol (trademark registered) manufactured by Helena Chemical Company), *Rhizopogon fulvigleba* (e.g., Myco-Sol (trademark registered) manufactured by Helena Chemical Company), *Saccharomyces cerevisiae* (e.g., a product manufactured by Lesaffre et Compagnie, FR), *Sclerotinia minor* (e.g., Sarritor (trademark registered) manufactured by Agrium Advanced Technologies), *Serratia entomophila* (e.g., Invade (trademark registered) manufactured by Wrightson Seeds), *Sporothrixinsectorum* (e.g., Sporothrix Es (trademark registered) manufactured by Biocerto, BR), *Steinernema carpocapsae* (e.g., Biosafe (trademark registered) manufactured by SDS Biotech K.K.), *Steinernema kushidai* (e.g., Shibaichi-Nema manufactured by Kubota Corporation), *Steinernema glaseri* (e.g., Biotopia (trademark registered) manufactured by Arysta LifeScience Corporation), *Streptomyces acidiscabies* (e.g., MBI-005EP (trademark registered) manufactured by Marrone Bioinnovations, CA using strain RL-110T), *Streptomyces candidus* (e.g., BioBac (trademark registered) manufactured by Biontech, TW using strain Y21007-2),

*Streptomyces galbus* (e.g., Mycostop (trademark registered) manufactured by Verdera using strain K61), *Streptomyces lydicus* (e.g., ACTINOVATE (trademark registered) manufactured by Natural Industries, US using strain WYEC108), *Streptomyces saraceticus* (e.g., Clanda (trademark registered) manufactured by A & A Group (Agro Chemical Corp.)), *Talaromyces flavus* (e.g., Momi-keeper (trademark registered) manufactured by Central Glass Co., Ltd. using strain B-422, Tough Block (trademark registered) manufactured by Idemitsu Kosan Co., Ltd. using strain SAY-Y-94-01, PROTUS (trademark registered) WG manufactured by Prophyta, DE using strain V117b), *Trichoderma asperellum* (e.g., a product manufactured by Isagro using strain ICC 012, T34 Biocontrol (trademark

registered) manufactured by Bioncontrol Technologies, ES using strain T34, EcoHope (trademark registered) manufactured by KUMIAI CHEMICAL INDUSTRY Co., Ltd. using strain SKT-1), *Trichoderma atroviride* (e.g., Esquive (trademark registered) WP manufactured by Agrauxine, FR, Tenet (trademark registered) manufactured by Agrimm Technologies Ltd, NZ or SENTINEL (trademark registered) each using strain LC52, EcoHope DJ (trademark registered) manufactured by KUMIAI CHEMICAL INDUSTRY Co., Ltd. using strain SKT-1), *Trichoderma gamsii* (e.g., BIO-TAM (trademark registered) manufactured by Bayer CropScience LP, US),
*Trichoderma harzianum* (e.g., T-Gro 7456 (trademark registered) manufactured by Dagutat Biolab using strain DB 103, Trianum-P (trademark registered) manufactured by Koppert using strain ITEM908, Trichoplus (trademark registered) manufactured by Biological Control Products, SA using strain KD, ROOT PRO (trademark registered) manufactured by Mycontrol Ltd. using strain TH-35), *Trichoderma harzianum* Rifai (e.g., PLANTSHIELD T-22G (trademark registered) manufactured by Firma BioWorks Inc, US using strain T-22, TRICHODEX (trademark registered) manufactured by Makhteshim Ltd, US using strain T-39), *Trichoderma lignorum* (e.g., Mycotric (trademark registered) manufactured by Futureco Bioscience, ES using strain TL-0601), *Trichodermapolysporum* (e.g., Binab TF WP (trademark registered) manufactured by BINAB Bio-Innovation AB, Sweden), *Trichoderma stromaticum* (e.g., TRICOVAB (trademark registered) manufactured by Ceplac, Brazil), *Trichoderma virens* (e.g., SOILGARD (trademark registered) manufactured by Certis LLC, US using strain GL-21),
*Trichoderma viride* (e.g., REMEDIER (trademark registered) WP manufactured by Isagro Ricerca, ITALIA using strain ICC080, Trianum-P (trademark registered) manufactured by Koppert using strain TV1), *Tsukamurella paurometabola* (e.g., HeberNem (trademark registered) using strain C-924), *Ulocladium oudemansii* (e.g., Botry-Zen (trademark registered) manufactured by Botry-Zen Ltd, NZ using strain HRU3 or BotryStop using strain U3 manufactured by BioWorks Inc.), *Variovorax paradoxus* (e.g., Fieldkeeper WP (trademark registered) manufactured by Central Glass using strain CGF452 6), Vesicular-Arbuscular (VA) mycorrhizal fungi (e.g., Dr. Kinkon (trademark registered) manufactured by Idemitsu Agri), *Verticillium alboatrum* (e.g., Dutch Trig (trademark registered) manufactured by Tree Care Innovations using strain WCS850), *Verticillium lecanii* (e.g., Vertalec (trademark registered) manufactured by Arysta LifeScience Corporation using strain IMI 179172, Mycotal (trademark registered) manufactured by Arysta LifeScience Corporation using strain IMI 263817), *Xanthomonas campestris* (e.g., Camperico L (trademark registered) manufactured by Taki Chemical), *Xanthomonas campestris* pv. *poae, Heterorhabditis bacteriophora, Steinernema feltiae, Steinernema kraussei, Steinernema riobrave, Steinernema scapterisci*; or insecticidal and microbicidal strains selected from mutants of the above-listed strains that each have its original distinguishing characteristics, and metabolites that are produced by the above-listed strains and active against plant pathogenic microbes.

[Examples]

As typical examples of the present invention, production examples, formulation examples, and test examples are recited below without limiting the present invention thereto.
A production example is shown below.

Production Example

Production of (S)-N-(1-(1-(6-trifluoromethylpyrimidin-4-yl)-1H-1,2,4-triazol-5-yl)ethyl)-3,5-bis(trifluoromethyl)benzamide (present inventive compound)

[Chem 3]

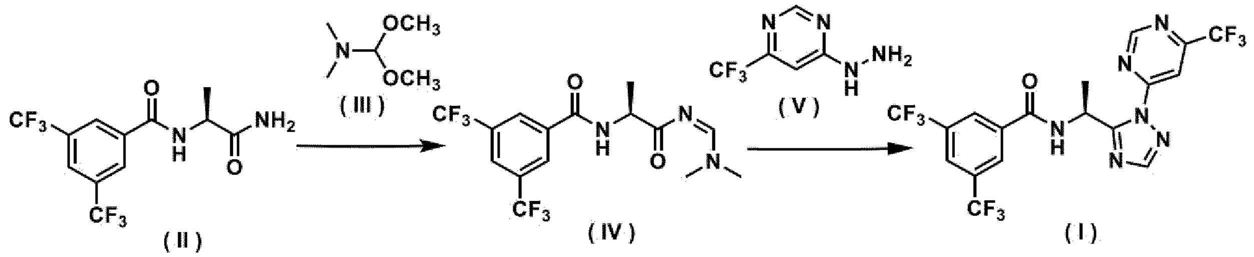

(S)-N-(2-amino-1-methyl-2-oxoethyl)-3,5-bis(trifluoromethyl)benzamide (400 mg (1.22 mmol)) was suspended in 10 mL of chloroform, and 218 mg (1.82 mmol) of N,N-dimethylformamide dimethyl acetal was added, and the mixture

was heated and stirred at 50 °C for 1 hour. The solvent was distilled off from the reaction solution under reduced pressure to obtain a crude compound represented by formula (IV). After adding 4 mL of dioxane and 4 mL of acetic acid to the obtained crude compound represented by formula (IV) and homogenizing, 239 mg (1.34 mmol) of 4-hydrazino-6-(trifluoromethyl)pyrimidine was added in three divisional portions at room temperature while stirring. After stirring the reaction solution at 50 °C for 10 hours, the solvent was distilled off under reduced pressure. 30 mL of ethyl acetate was added to the residue, and after washing with 10 mL of saturated aqueous sodium bicarbonate, the ethyl acetate layer was dried over magnesium sulfate. After filtering off the desiccant, the solvent was distilled off from the filtrate under reduced pressure. 5 mL of n-hexane/ethyl acetate mixed solvent (volume ratio 4/1) was added to the residue, and the precipitated crystals were filtered and washed with 5 mL of n-hexane to obtain the compound of the present inventive 516 mg of (S)-N-(1-(1-(6-trifluoromethylpyrimidin-4-yl)-1H-1,2,4-triazol-5-yl)ethyl)-3,5-bis(trifluoromethyl)benzamide was obtained.

Yield: 85%
Physical property: Melting point 162-164 °C

Reference Production Example

Production of 4-hydrazino-6-(trifluoromethyl)pyrimidine

4-hydrazino-6-(trifluoromethyl)pyrimidine, a known compound, which was used in the above-mentioned Production Example 1, was prepared in the following method.

[Chem 4]

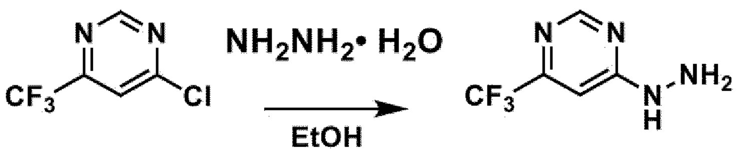


3.29 g (65.7 mmol) of hydrazine hydrate was added to 10 mL of ethanol while stirring, and the mixture was made to 0 °C in an ice bath. 4.00 g (21.9 mmol) of 4-chloro-6-(trifluoromethyl)pyrimidine was slowly added dropwise while stirring. After the dropwise addition was completed, the mixture was stirred at room temperature for 12 hours, and half of the ethanol was distilled off under reduced pressure. The precipitated solid was filtered and washed with 5 mL of cold water and 5 mL of cold ethanol to obtain 2.18 g of the desired 4-hydrazino-6-(trifluoromethyl)pyrimidine (yield: 56%).
Formulation examples of the present agrohorticultural pest control agent are presented below. In the formulation examples, the term parts denotes "parts by mass".

Formulation Example 1

| | |
|---|---|
| Present inventive compound | 10 parts |
| Xylene | 70 parts |
| N-methylpyrrolidone | 10 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

Formulation Example 2

| | |
|---|---|
| Present inventive compound | 3 parts |
| Clay powder | 82 parts |

(continued)

| | |
|---|---|
| Diatomaceous earth powder | 15 parts |

A dust was prepared by mixing uniformly and grinding the above ingredients.

Formulation Example 3

| | |
|---|---|
| Present inventive compound | 5 parts |
| Mixed powder of bentonite and clay | 90 parts |
| Calcium lignin sulfonate | 5 parts |

Granules were prepared by mixing the above ingredients uniformly, and kneading the resulting mixture together with a suitable amount of water, followed by granulation and drying.

Formulation Example 4

| | |
|---|---|
| Present inventive compound | 20 parts |
| Mixture of kaolin and synthetic high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

Test Examples are presented below. As a comparison compound, (S)-N-(1-(1-(pyrimidin-2-yl)-1H-1,2,4-triazol-5-yl)ethyl)-3,5-bis(trifluoromethyl)benzamide was used. Said compound is described as No. 66 in Patent Document 1 and as No. I-66 in Patent Document 2, having the following structural formula.

Test Example 1.

Control value test against the green peach aphid (Myzus persicae)

Chinese cabbage was planted in plastic pots with a diameter of 8 cm and a height of 8 cm to breed green peach aphids, and the number of the infesting aphids in each pot was investigated. The present inventive compound was dispersed in water and diluted to a 500 ppm chemical solution, and the chemical solution was sprayed on the stems and leaves of the Chinese cabbage plants planted in the pots. After air drying, the pots were kept in a greenhouse. On the 6th day after the spray of the chemical solution, the number of the green peach aphids infesting on each Chinese cabbage plant was counted and the control value was calculated on the following equation, whereby the effect was judged according to the criterion below.

The Comparative test compound was tested in the same way, and the control value was calculated and judged.

As a result of this test, the present inventive compound and the comparative compound showed an activity of B or higher.

[Math 1]

$$\text{Control value} = 100 - \{(T \times Ca)/(Ta \times C)\} \times 100$$

Ta: number of infestants before spraying in the treated group,
T: number of infestants after spraying in the treated group,
Ca: number of infestants before spraying in the untreated group,
C: number of infestants after spraying in the untreated group.

Criterion

A: Control value 100%
B: Control value 99 - 90%
C: Control value 89 - 80%
D: Control value 79 - 50%

Test Example 2. Insecticidal test against the smaller brown planthopper (Laodelphax striatellus)

The present inventive compound was dispersed in water to adjust the concentration to 500 ppm and a rice seedling was immersed therein for 30 seconds; after air dried, the seedling was put in a test tube, 10 third instar nymphs were inoculated and the test tube was sealed with a cotton plug. Eight days after, the alive and dead insects were counted to calculate the corrected mortality according to the following equation and the insecticidal effect was judged according to the criterion shown below.
The Comparative test compound was tested in the same way, and the corrected mortality was calculated and judged.
As a result of this test, the present inventive compound and the comparative compound showed an activity of B or higher.

[Math 2]

$$\text{Corrected mortality (\%)} = \frac{(\text{Survival rate in untreated group} - \text{Survival rate in treated group})}{(\text{Survival rate in untreated group})} \text{x}100$$

Criterion:

A: Corrected mortality 100%
B: Corrected mortality 99% - 90%
C: Corrected mortality 89% - 80%
D: Corrected mortality 79% - 50%

Test Example 3. Insecticidal test against the diamond back moth (Plutella xylostella)

The adult diamond back moths were released and allowed to oviposit on a Chinese cabbage seedling. Two days after the release, the seedling having the eggs deposited thereon was immersed for about 30 seconds in a liquid chemical prepared by diluting a preparation containing the present inventive compound as an active ingredient to adjust the concentration to 500 ppm. After air dried, it was allowed to stand in a room thermostatted at 25 °C. Six days after the immersion, the alive insects were counted. The corrected mortality was calculated according to the following equation and the insecticidal effect was judged according to the criterion shown below. The test was carried out with triplicate groups.
The Comparative test compound was tested in the same way, and the corrected mortality was calculated and judged.
As a result of this test, the present inventive compound and the comparative compound showed an activity of B or higher.

[Math 3]

$$\text{Corrected mortality (\%)} = \frac{(\text{Survival rate in untreated group} - \text{Survival rate in treated group})}{(\text{Survival rate in untreated group})} \text{x}100$$

Criterion:

A: Corrected mortality 100%
B: Corrected mortality 99% - 90%
C: Corrected mortality 89% - 80%
D: Corrected mortality 79% - 50%

Test Example 4. Insecticidal test against the common cutworm (Spodoptera litura)

Apiece of cabbage leaf (cultivar; Shikidori) was dipped for about 30 seconds in a chemical solution prepared by diluting a preparation containing the present inventive compound as an active ingredient to adjust the concentration to 500 ppm. After air dried, it was placed in a plastic Petri dish with a diameter of 9 cm and inoculated with second-instar larvae of common cutworm, after which the dish was closed and then allowed to stand in a room thermostatted at 25 °C. Eight days after the inoculation, the number of the alive insects were counted. The corrected mortality was calculated according to the following equation and the insecticidal effect was judged according to the following criterion. The test was carried out with triplicate groups of 10 insects.

The Comparative test compound was tested in the same way, and the corrected mortality was calculated and judged.

As a result of this test, the present inventive compound and the comparative compound showed an activity of B or higher.

[Math 4]

$$\text{Corrected mortality (\%)} = \frac{\text{(Survival rate in untreated group} - \text{Survival rate in treated group)}}{\text{(Survival rate in untreated group)}} \times 100$$

Criterion:

A: Corrected mortality 100%
B: Corrected mortality 99% - 90%
C: Corrected mortality 89% - 80%
D: Corrected mortality 79% - 50%

Test Example 5. Safety test for the Western honeybee (oral acute toxicity test)

Seven to ten adult Western honeybees (Apis mellifera) were released in a plastic cage (8 cm x 12 cm x 20 cm), and after fasting for 2 hours, they were oral administered, using a 1.5 ml microtube, with a 50% sucrose solution as a test bait containing a chemical solution of a predetermined concentration. Four hours after the administration, the test bait was taken out, and the amount of the compound taken per animal was calculated from the remaining amount. At the same time, the animals were fed with a sufficient amount of 50% sucrose solution, maintained at 25°C, H55-75%, 16L8D. The numbers of dead and abnormal insects were counted after 24, 48 and 96 hours to calculate the median lethal dose ($LD_{50}$ value). The untreated group was fed with a 50% sucrose solution with chemical and was maintained under the same conditions as the chemical-treated group.

As a result of this test, the $LD_{50}$ value of the present inventive compound for one adult honeybee after 96 hours was 2.04 μg, and the $LD_{50}$ value of the comparative compound was 0.007 to 0.01 μg. There were no dead insects or abnormal insects in the untreated group even after 96 hours. The results of this test indicate that the present inventive compound is improved in its safety for the Western honeybee more than 200 times compared with the comparative compound.

The results in the above-mentioned Test Examples 1 to 5 revealed that the present inventive compound is safe for honeybees and has an excellent effect against agricultural or horticultural pests.

[Industrial Applicability]

The pyrimidinyl triazole compound or a salt thereof can be applied as an agrohorticultural pest control agent safe for honeybees.

## Claims

1. A compound represented by formula (I) or a salt thereof:

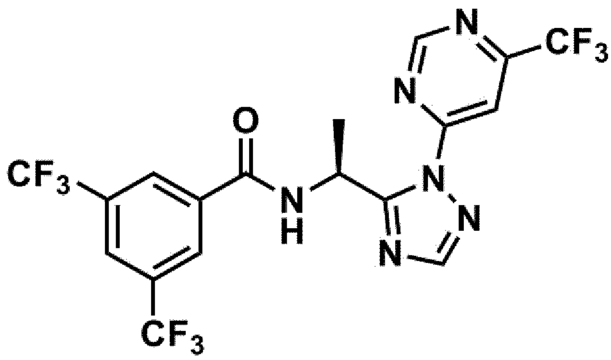

(I)

2. An agrohorticultural pest control agent comprising the compound or a salt thereof according to claim 1 as an active ingredient.

3. A method of use of an agrohorticultural pest control agent, **characterized in that** an effective amount of the agrohorticultural pest control agent according to claim 2 is treated on a target plant or soil.

4. A method of controlling an agrohorticultural pest, **characterized in that** an effective amount of the agrohorticultural pest control agent according to claim 2 is treated on a target plant or soil.

## INTERNATIONAL SEARCH REPORT

International application No.
**PCT/JP2022/037582**

**A. CLASSIFICATION OF SUBJECT MATTER**

***C07D 403/04***(2006.01)i; ***A01P 7/04***(2006.01)i; ***A01N 43/653***(2006.01)i
FI: C07D403/04 CSP; A01P7/04; A01N43/653 K

According to International Patent Classification (IPC) or to both national classification and IPC

**B. FIELDS SEARCHED**

Minimum documentation searched (classification system followed by classification symbols)

C07D403/00-403/14; A01P7/00-7/04; A01N43/00-43/92

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2017/192385 A1 (ELANCO TIERGESUNDHEIT AG) 09 November 2017 (2017-11-09) whole document | 1–4 |
| A | WO 2019/170626 A1 (BAYER AKTIENGESELLSCHAFT) 12 September 2019 (2019-09-12) whole document | 1–4 |
| A | WO 2019/201835 A1 (BAYER AKTIENGESELLSCHAFT) 24 October 2019 (2019-10-24) whole document | 1–4 |
| A | WO 2019/202077 A1 (BAYER AKTIENGESELLSCHAFT) 24 October 2019 (2019-10-24) whole document | 1–4 |
| A | WO 2020/002563 A1 (SYNGENTA PARTICIPATIONS AG) 02 January 2020 (2020-01-02) whole document | 1–4 |
| A | WO 2020/094363 A1 (SYNGENTA PARTICIPATIONS AG) 14 May 2020 (2020-05-14) whole document | 1–4 |
| P, A | WO 2021/224323 A1 (BAYER AKTIENGESELLSCHAFT) 11 November 2021 (2021-11-11) whole document | 1–4 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 November 2022** | **06 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/037582**

| Patent document cited in search report | Publication date (day/month/year) | Patent family member(s) | Publication date (day/month/year) |
|---|---|---|---|
| WO 2017/192385 A1 | 09 November 2017 | JP 2018-517716 A | |
| | | US 2018/0186778 A1 entire document | |
| | | US 2019/0256501 A1 | |
| | | EP 3452460 A1 | |
| | | KR 10-2018-0132100 A | |
| | | CN 109311841 A | |
| WO 2019/170626 A1 | 12 September 2019 | JP 2021-516238 A | |
| | | US 2020/0404919 A1 entire document | |
| | | EP 3761791 A1 | |
| | | CN 111818801 A | |
| | | KR 10-2020-0129128 A | |
| WO 2019/201835 A1 | 24 October 2019 | JP 2021-521223 A | |
| | | US 2021/0155608 A entire document | |
| | | EP 3781553 A1 | |
| | | EP 4039682 A1 | |
| | | KR 10-2020-0143456 A | |
| | | CN 112189011 A | |
| WO 2019/202077 A1 | 24 October 2019 | JP 2021-522181 A | |
| | | US 2022/0002268 A1 entire document | |
| | | EP 3820861 A1 | |
| | | CN 112135819 A | |
| | | KR 10-2021-0003154 A | |
| WO 2020/002563 A1 | 02 January 2020 | JP 2021-528473 A | |
| | | US 2021/0267203 A1 entire document | |
| | | EP 3814332 A1 | |
| | | CN 112351978 A | |
| | | KR 10-2021-0028201 A | |
| WO 2020/094363 A1 | 14 May 2020 | JP 2022-509019 A | |
| | | US 2021/0403462 A1 entire document | |
| | | EP 3877380 A1 | |
| | | CN 112955442 A | |
| | | TW 202024055 A | |
| WO 2021/224323 A1 | 11 November 2021 | TW 202208347 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017192385 A
- WO 2019170626 A
- WO 2019197468 A
- WO 2019201835 A
- WO 2019202077 A
- WO 2019206799 A
- WO 2019215198 A
- WO 2020002563 A
- WO 2020053365 A
- WO 2020079198 A
- WO 2020094363 A
- WO 2020182649 A
- WO 2020188014 A
- WO 2020188027 A
- WO 2020193341 A
- WO 2020212235 A
- WO 2021013719 A
- WO 2021013720 A
- WO 2021069567 A
- WO 2021069569 A
- WO 2021083936 A
- WO 2021099303 A
- WO 2021110891 A
- WO 2021122645 A
- EP 0374753 A
- WO 9307278 A
- WO 9534656 A
- EP 0427529 A
- EP 451878 A
- WO 03052073 A


### Non-patent literature cited in the description


- Proceedings of the National Academy of Sciences of the United States of America. *Proc. Natl. Acad. Sci. USA,* 1990, vol. 87, 7175-7179
- **WEED.** *Science,* 2005, vol. 53, 728-746
- **GURA T.** Repairing the Genome's Spelling Mistakes. *Science,* 1999, vol. 285, 316-318